# EUROPEAN PATENT APPLICATION

(11) **EP 3 091 061 A2**
(43) Date of publication of application: **09.11.2016**
(21) Application number: 16168292.7
(22) Date of filing: 04.05.2016
(51) Int. Cl.: C09K 19/04, C09K 19/30, C09K 19/12

(54) **LIQUID CRYSTAL COMPOSITION AND LIQUID CRYSTAL DISPLAY INCLUDING THE SAME**

(30) Priority: 07.05.2015 KR 20150063992
(71) Applicant: Samsung Display Co., Ltd., Gyeonggi-do 17113 (KR)
(72) Inventor: Ahn, Byung Wook, 201-406 Seoul (KR); Takeshita, Fusayuki, 204-1502 Seoul (KR); Oh, Keun Chan, 131-2302 Gyeonggi-do (KR)
(74) Representative: Dr. Weitzel & Partner

(57) **Abstract**

A liquid crystal composition includes at least one liquid crystal molecule having a positive dielectric anisotropy represented by Chemical Formula 1:

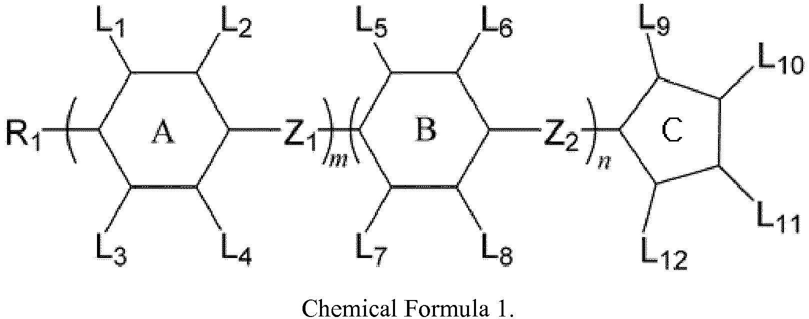

In Chemical Formula 1, each of A and B is independently 1, 4-cyclohexylene or 1, 4-phenylene, C is cyclopentyl or cyclopentadienyl, each of Z₁ and Z₂ is independently a single bond, alkylene group having the number of carbons from 1 to 10, carbonyloxy, -CF₂O-, -OCF₂-, -CH₂O-, or -OCH₂-, each of L₁ to L₄ is independently dihydro group when A is 1, 4-cyclohexylene, each of L₁ to L₄ is independently -H, -F, or -CF₃ when A is 1, 4-phenylene, each of L₅ to L₈ is independently dihydro group when B is 1, 4-cyclohexylene, each of L₅ to L₈ is independently -H, -F, or -CF₃ when B is 1, 4-phenylene, each of L₉ to L₁₂ is independently dihydro group, difluoro group, or two group including one hydrogen and one fluorine when C is cyclopentyl, and each of L₉ to L₁₂ is independently -H, -F, or -CF₃ when C is cyclopentadienyl, each of m and n is 0 to 2, provided that m and n are not simultaneously 0, and R₁ is an alkyl group or an alkoxy group, and the number of carbons of R₁ is about 1 to about 10.

## Description

### BACKGROUND

### (a) Field

The present disclosure relates to a liquid crystal composition and a liquid crystal display including the same.

### (b) Description of the Related Art

A liquid crystal display is currently one of the most used flat panel displays. A liquid crystal display is generally configured with two sheets of display panels facing each other, a liquid crystal layer provided therebetween, and a field generating electrode such as a pixel electrode or a common electrode provided on at least one of the display panels.

The liquid crystal display applies a voltage to the field generating electrode to generate an electric field on the liquid crystal layer, determines an alignment of liquid crystal molecules provided in the liquid crystal layer, and controls transmittance of light passing through the liquid crystal layer.

The liquid crystal composition employed in the liquid crystal display is very important to achieving a desired image by controlling the transmittance of light. In particular, with various uses of liquid crystal displays, various characteristics such as low-voltage driving, a high voltage holding ratio ("VHR"), a wide viewing angle, a wide operation temperature range, high-speed response, and high transmittance are desirable in order to meet industry demands and/or performance standards.

The above information disclosed in this Background section is only to enhance the understanding of the background of the disclosure and therefore it may contain information that does not form the prior art that is already known in this country to a person of ordinary skill in the art.

### SUMMARY

The present disclosure has been made in an effort to provide a liquid crystal composition and a liquid crystal display including the same that have a particular chemical component and a positive dielectric anisotropy.

In an exemplary embodiment, a liquid crystal composition includes at least one liquid crystal molecule having a positive dielectric anisotropy, as represented by Chemical Formula 1:

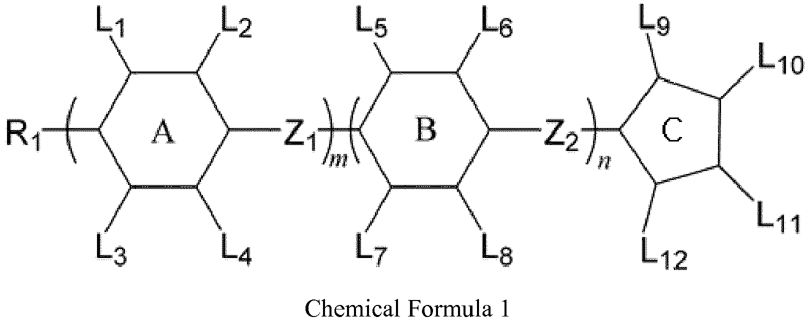

In an aspect of the exemplary embodiment, in Chemical Formula 1, each of A and B is independently 1, 4-cyclohexylene or 1, 4-phenylene, C is cyclopentyl or cyclopentadienyl, each of Z₁ and Z₂ is independently a single bond, alkylene group having the number of carbons from 1 to 10, carbonyloxy, -CF₂O-, -OCF₂-, -CH₂O-, or -OCH₂-,
each of L₁ to L₄ is independently dihydro group when A is 1, 4-cyclohexylene, each of L₁ to L₄ is independently -H, -F, or -CF₃ when A is 1, 4-phenylene, each of L₅ to L₈ is independently dihydro group when B is 1, 4-cyclohexylene, each of L₅ to L₈ is independently -H, -F, or -CF₃ when B is 1, 4-phenylene, each of L₉ to L₁₂ is independently dihydro group, difluoro group, or two group including one hydrogen and one fluorine when C is cyclopentyl, and each of L₉ to L₁₂ is independently -H, -F, or -CF₃ when C is cyclopentadienyl,
each of m and n is 0 to 2, provided that m and n are not simultaneously 0, and R₁ is an alkyl group or an alkoxy group. Herein, the number of carbons of R₁ may be about 1 to about 10. Chemical Formula 1 may include a difluoro (diF) group substituted cyclopentyl group.

In another exemplary embodiment, Chemical Formula 1 may be represented by at least one of Chemical Formulas 1-1 to Chemical Formula 1-16:

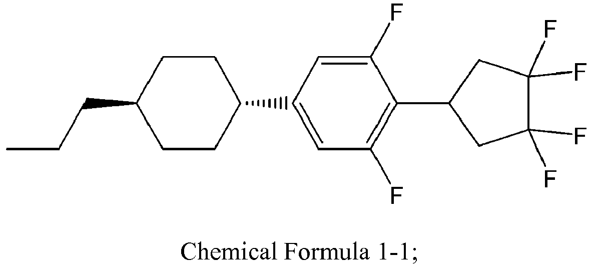

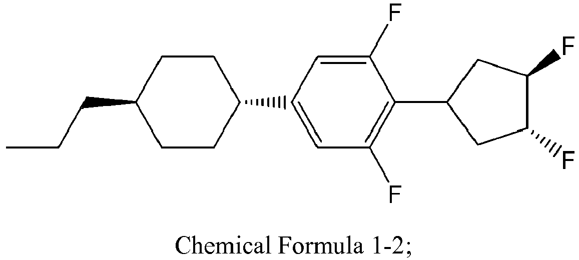

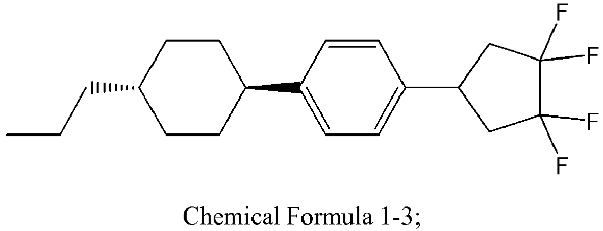

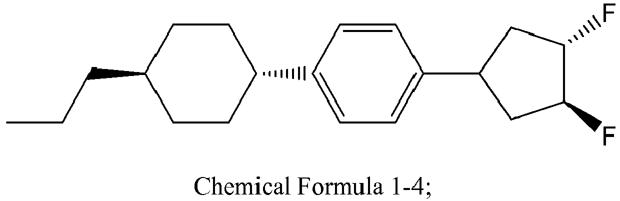

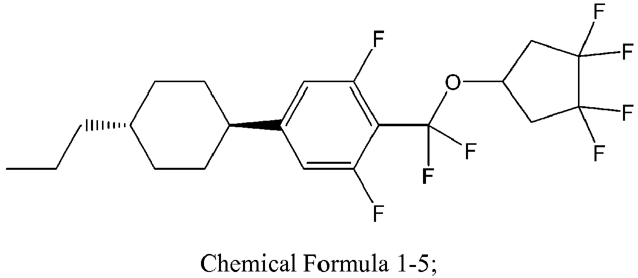

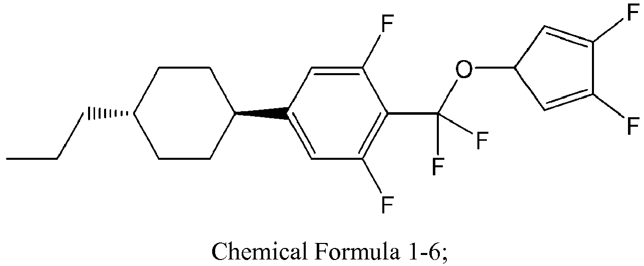

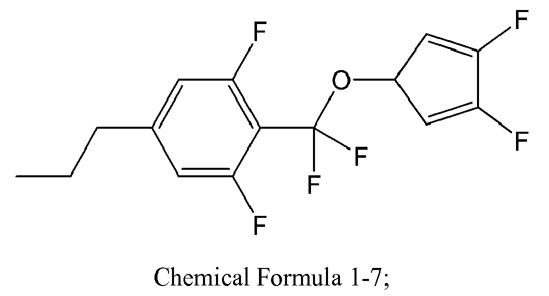

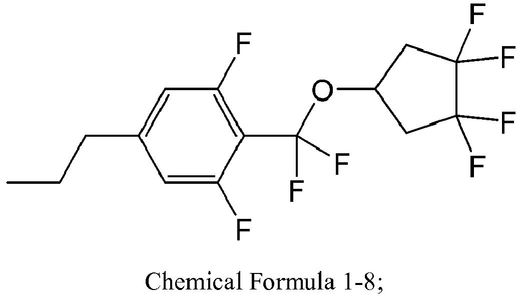

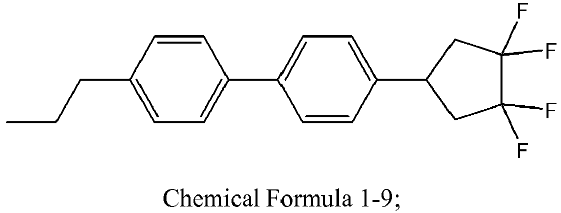

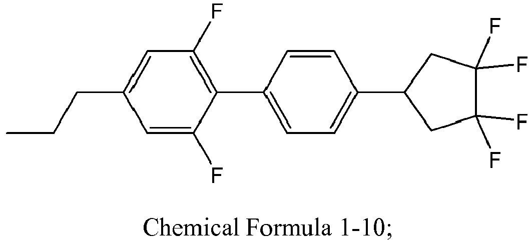

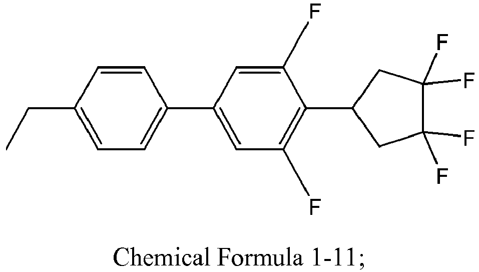

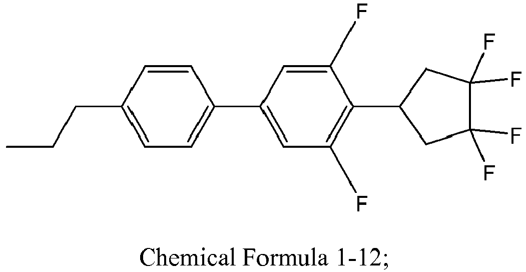

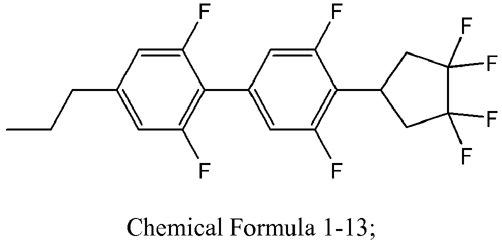

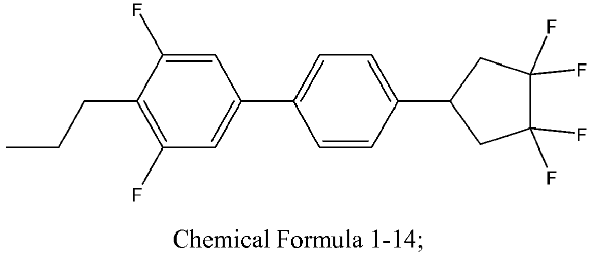

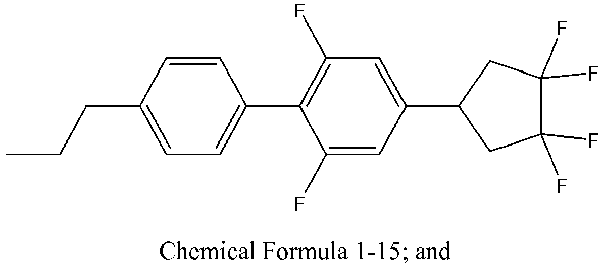

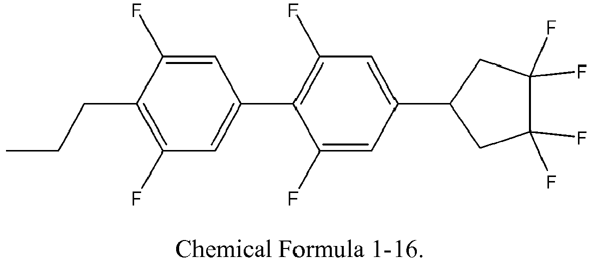

In still another exemplary embodiment, the liquid crystal composition may further include at least one selected from a group consisting of liquid crystal molecules represented by Chemical Formula 2 and Chemical Formula 3:

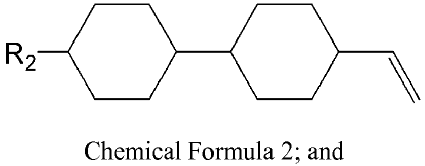

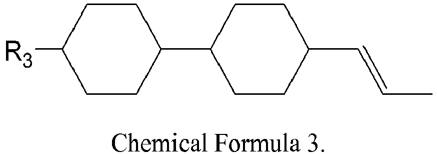

In an aspect of the exemplary embodiment, in Chemical Formula 2 and Chemical Formula 3, each of R₂ and R₃ may be independently an alkyl group or an alkoxy group. Herein, the number of carbons of each of R₂ and R₃ may be about 1 to about 10.

In another exemplary embodiment, the liquid crystal composition may further include at least one selected from a group consisting of liquid crystal molecules represented by Chemical Formula 4, Chemical Formula 5 and Chemical Formula 6:

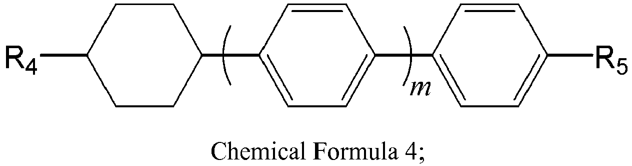

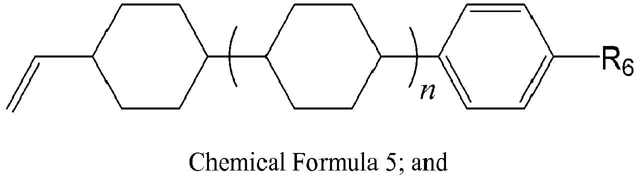

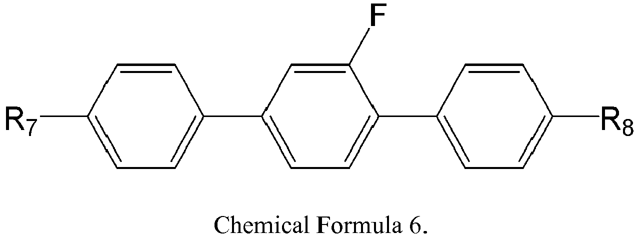

In an aspect of the exemplary embodiment, in Chemical Formula 4, Chemical Formula 5 and Chemical Formula 6, each of R₄ to R₈ may be independently an alkyl group or an alkoxy group, and each of m and n may be about 0 to about 2. Herein, the number of carbons of each of R₄ to R₈ may be about 1 to about 10.

In another exemplary embodiment, the liquid crystal composition may further include at least one selected from a group consisting of liquid crystal molecules represented by Chemical Formula 7, Chemical Formula 8, Chemical Formula 9 and Chemical Formula 10:

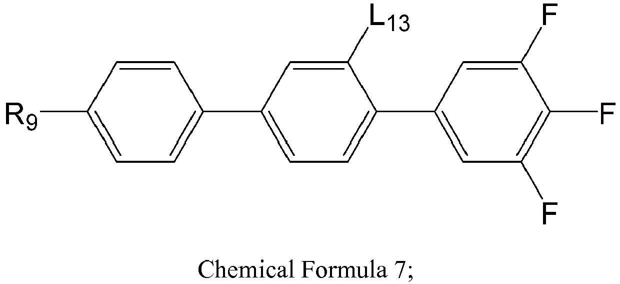

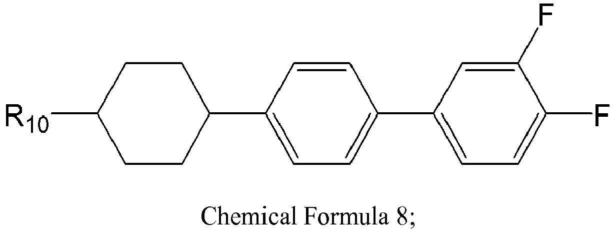

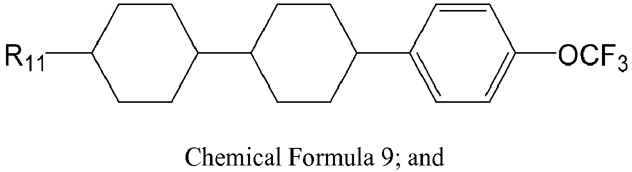

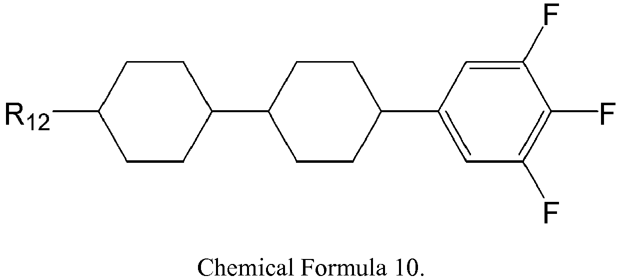

In an aspect of the exemplary embodiment, in Chemical Formula 7 to Chemical Formula 10, each of R₉ to R₁₂ may be independently an alkyl group or an alkoxy group, and L₁; may be H or halogen. Herein, the number of carbons of each of R₉ to R₁₂ may be about 1 to about 10.

In another exemplary embodiment, the liquid crystal composition may further include at least one selected from a group consisting of liquid crystal molecules represented by Chemical Formula 11, Chemical Formula 12 and Chemical Formula 13:

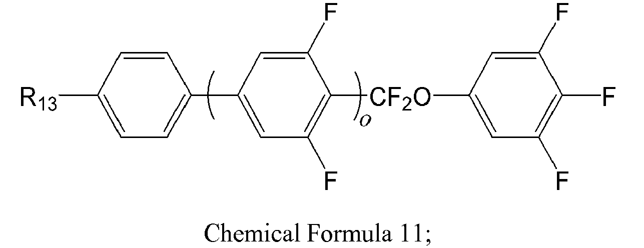

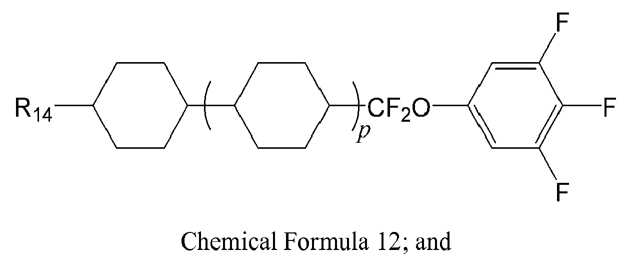

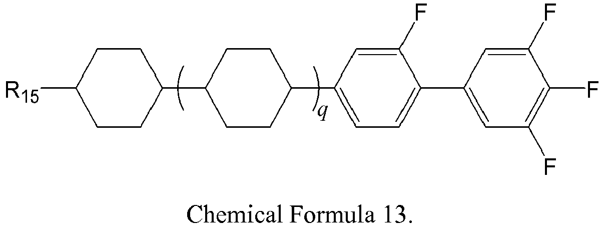

In an aspect of the exemplary embodiment, in Chemical Formula 11, Chemical Formula 12 and Chemical Formula 13, each of R₁₃ to R₁₅ may be independently an alkyl group or an alkoxy group, and each of o, p, q may be about 0 to about 2. Herein, the number of carbons of each of R₁₃ to R₁₅ may be about 1 to about 10.

In an exemplary embodiment, the at least one liquid crystal molecule represented by Chemical Formula 1 is present in an amount of about 0.1 weight percent (wt.%) to about 80 wt.%, based on a total weight of the liquid crystal composition.

In another exemplary embodiment, a liquid crystal display includes a first substrate; a first electrode and a second electrode that are disposed on the first substrate with an insulating layer therebetween; a second substrate facing the first substrate; and a liquid crystal layer disposed between the first substrate and the second substrate. In an aspect of the exemplary embodiment, the liquid crystal layer may include at least one liquid crystal molecule having a positive dielectric anisotropy represented by Chemical Formula 1:

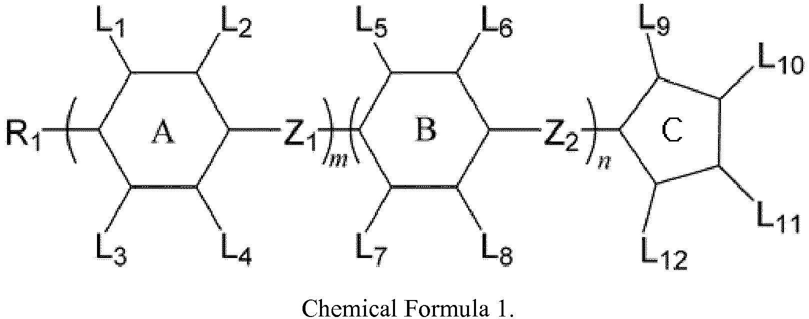

In another aspect of the exemplary embodiment, in Chemical Formula 1, each of A and B may be independently 1, 4-cyclohexylene or 1, 4-phenylene, C is cyclopentyl or cyclopentadienyl, each of Z₁ and Z₂ is independently a single bond, alkylene group having the number of carbons from 1 to 10, carbonyloxy, -CF₂O-, -OCF₂-, -CH₂O-, or -OCH₂-,
wherein each of L₁ to L₄ is independently dihydro group when A is 1, 4-cyclohexylene, each of L₁ to L₄ is independently -H, -F, or -CF₃ when A is 1, 4-phenylene, each of L₅ to L₈ is independently dihydro group when B is 1, 4-cyclohexylene, each of L₅ to L₈ is independently -H, -F, or -CF₃ when B is 1, 4-phenylene, each of L₉ to L₁₂ is independently dihydro group, difluoro group, or two group including one hydrogen and one fluorine when C is cyclopentyl, and each of L₉ to L₁₂ is independently -H, -F, or -CF₃ when C is cyclopentadienyl,
each of m and n may be 0 to 2, provided that m and n are not simultaneously 0, and R₁ may be an alkyl group or an alkoxy group. Herein, the number of carbons of R₁ may be 1 to 10. Chemical Formula 1 may include a difluoro (diF) group substituted cyclopentyl group.

In another exemplary embodiment, Chemical Formula 1 may be represented by at least one of the following Chemical Formula 1-1 to Chemical Formula 1-16:

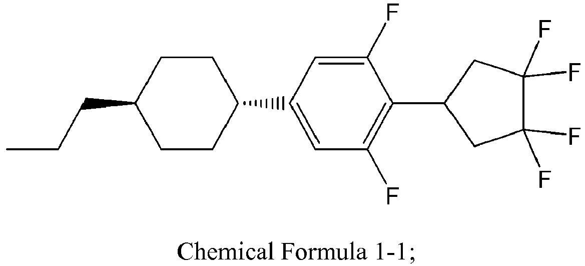

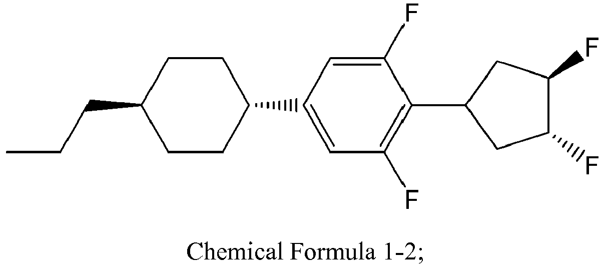

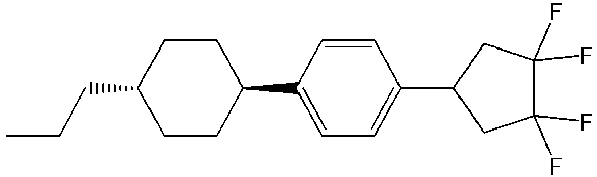

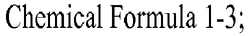

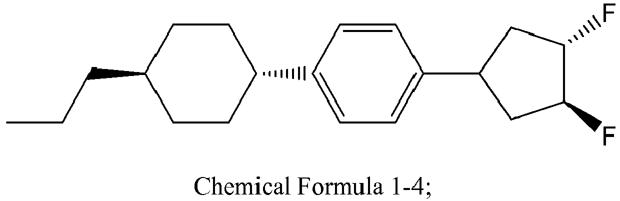

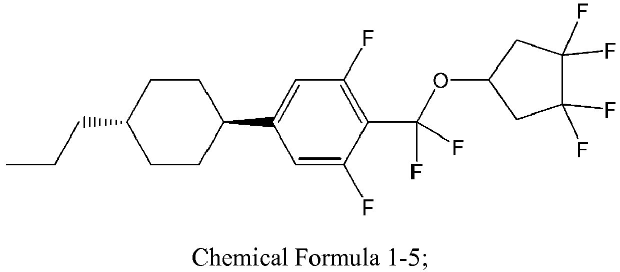

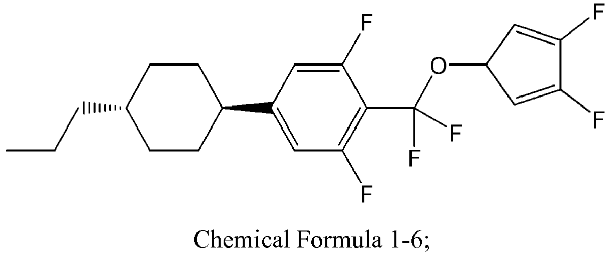

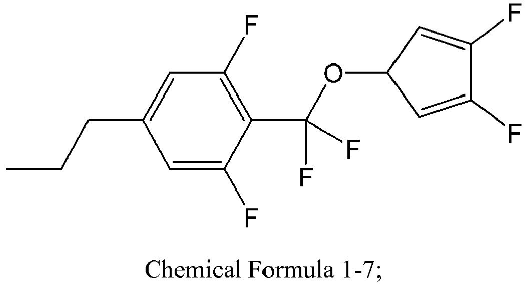

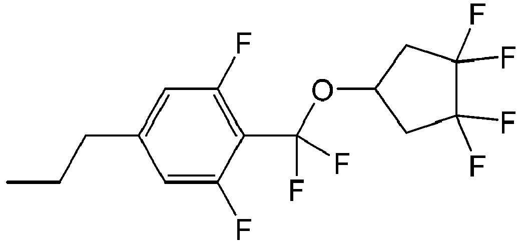

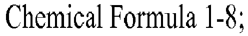

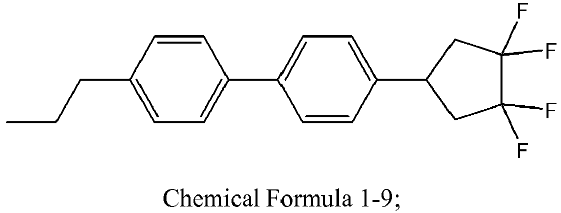

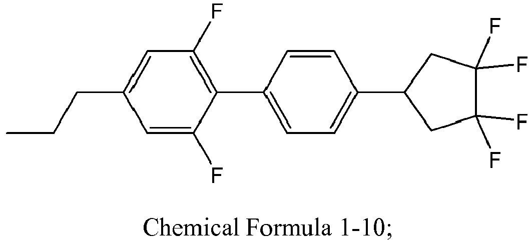

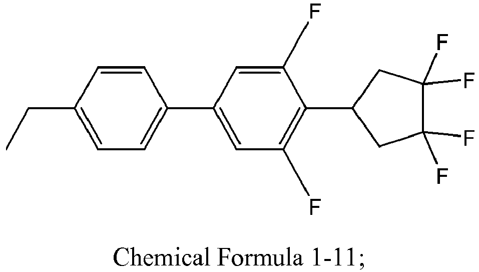

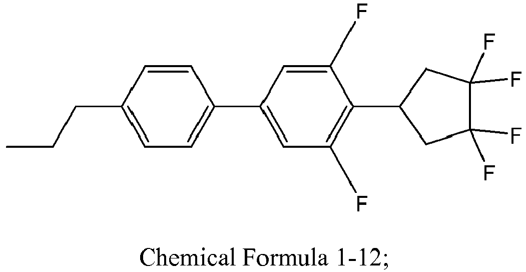

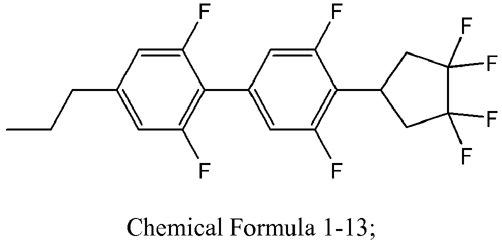

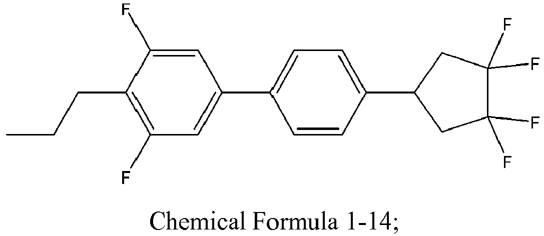

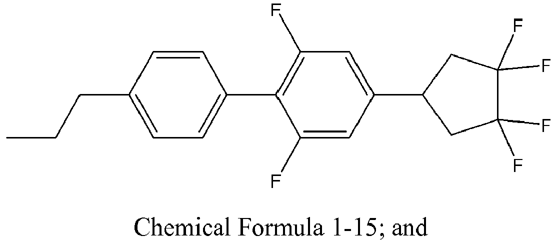

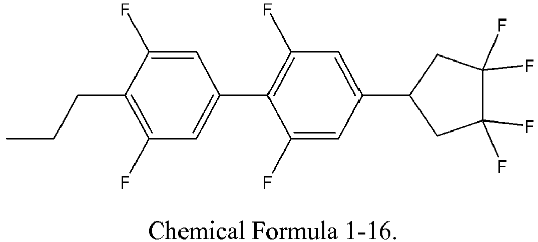

In still another exemplary embodiment, the liquid crystal layer may further include at least one selected from a group consisting of liquid crystal molecules represented by Chemical Formula 2 and Chemical Formula 3:

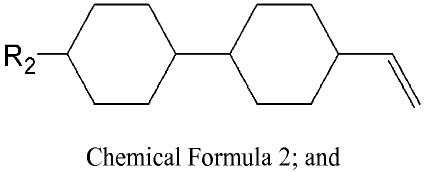

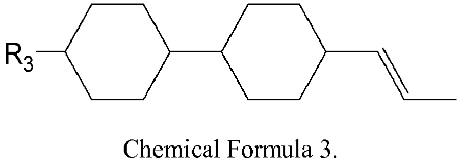

In an aspect of the exemplary embodiment, in Chemical Formula 2 and Chemical Formula 3, each of R₂ and R₃ may be independently an alkyl group or an alkoxy group. Herein, the number of carbons of each of R₂ and R₃ may be about 1 to about 10.

In another exemplary embodiment, the liquid crystal layer may further include at least one selected from a group consisting of liquid crystal molecules represented by Chemical Formula 4, Chemical Formula 5 and Chemical Formula 6:

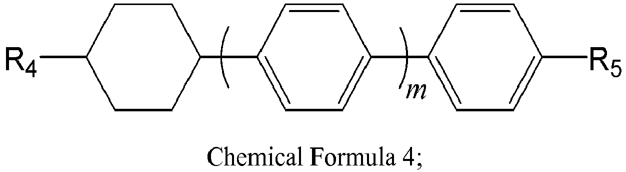

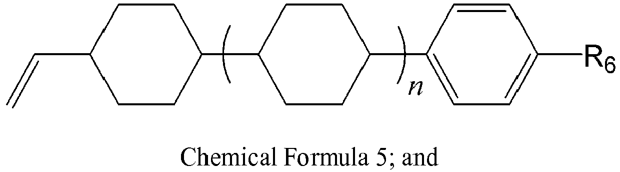

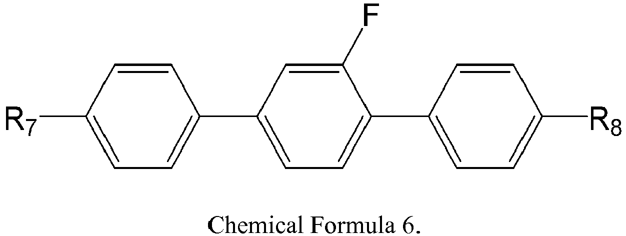

In an aspect of the exemplary embodiment, in Chemical Formula 4, Chemical Formula 5 and Chemical Formula 6, each of R₄ to R₈ may be independently an alkyl group or an alkoxy group, and each of m and n may be about 0 to about 2. Herein, the number of carbons of each of R₄ to R₈ may be about 1 to about 10.

In another exemplary embodiment, the liquid crystal layer may further include at least one selected from a group consisting of liquid crystal molecules represented by Chemical Formula 7, Chemical Formula 8, Chemical Formula 9 and Chemical Formula 10:

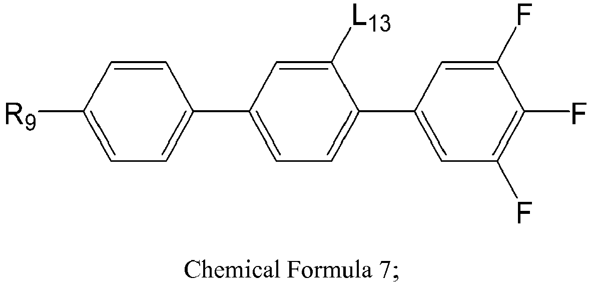

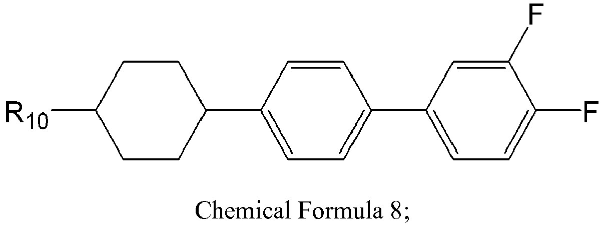

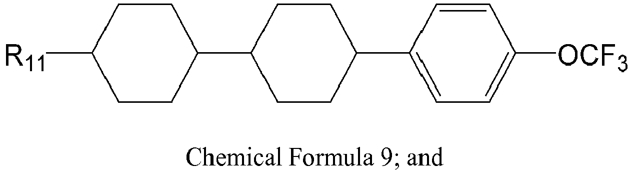

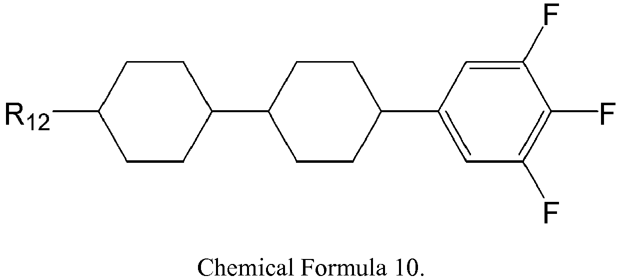

In an aspect of the exemplary embodiment, in Chemical Formula 7 to Chemical Formula 10, each of R₉ to R₁₂ may be independently an alkyl group or an alkoxy group, and L₁₃ may be hydrogen or halogen. Herein, the number of carbons of each of R₉ to R₁₂ may be about 1 to about 10.

In another exemplary embodiment, the liquid crystal layer may further include at least one selected from a group consisting of liquid crystal molecules represented by Chemical Formula 11, Chemical Formula 12 and Chemical Formula 13:

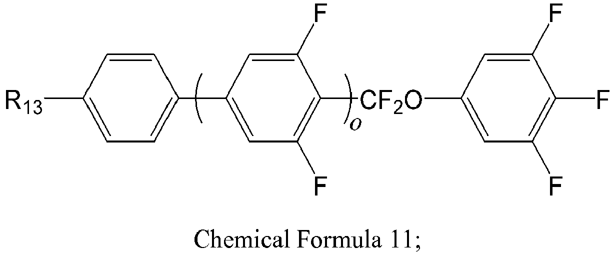

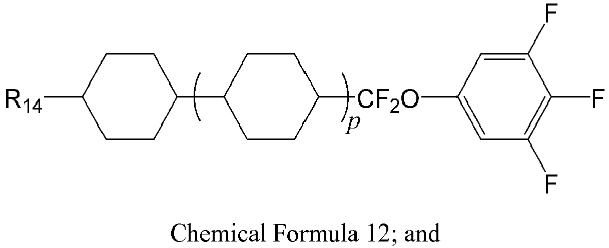

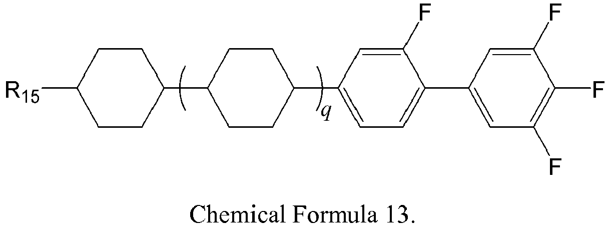

In an aspect of the exemplary embodiment, in Chemical Formula 11, Chemical Formula 12, and Chemical Formula 13, each of R₁₃ to R₁₅ may be independently an alkyl group or an alkoxy group, and each of o, p, q may be about 0 to about 2. Herein, the number of carbons of each of R₁₃ to R₁₅ is about 1 to about 10.

In another exemplary embodiment, the insulating layer may be disposed on the first electrode, the second electrode may be disposed on the insulating layer, the first electrode may be formed to have a one-plate shape, and the second electrode may include a plurality of branch electrodes.

In still another exemplary embodiment, the plurality of branch electrodes may overlap the first electrode of the one-plate shape.

In yet another exemplary embodiment, the second electrode may be connected to a thin film transistor through a contact hole penetrating through the insulating layer.

In another exemplary embodiment, liquid crystal molecules contained in the liquid crystal layer may tilt in a direction parallel to the branch electrodes when an electric field is not applied to the liquid crystal layer.

In still another exemplary embodiment, the liquid crystal display may further include an alignment layer disposed on the second electrode, and the alignment layer may be rubbed or photo-aligned in a direction parallel to the branch electrodes.

In yet another exemplary embodiment, the liquid crystal molecules may tilt in a direction parallel to the electric field when the electric field is applied to the liquid crystal layer.

In another exemplary embodiment, the first electrode may be formed to have a one-plate shape at an area corresponding to a unit pixel.

According to an embodiment of the present disclosure, a liquid crystal display may be formed with a liquid crystal composition having a particular component having positive dielectric anisotropy.

### BRIEF DESCRIPTION OF THE DRAWINGS

The above and other aspects, advantages and features of this disclosure will become more apparent by describing in further detail exemplary embodiments thereof with reference to the accompanying drawings, in which:
FIG. 1 illustrates a top plan view of an exemplary embodiment of a liquid crystal display;
FIG. 2 illustrates a cross-sectional view of FIG. 1 taken along line II-II;
FIG. 3 illustrates a schematic cross-sectional view illustrating an exemplary embodiment of an alignment of liquid crystal molecules depending on whether an electric field is applied in a liquid crystal display; and
FIG. 4 illustrates a schematic cross-sectional view of an exemplary embodiment of an in-plane switching mode liquid crystal display as an exemplary variation of FIG. 3.

### DETAILED DESCRIPTION OF THE EMBODIMENTS

The present disclosure will be described more fully hereinafter with reference to the accompanying drawings, in which exemplary embodiments of the invention are shown. However, it is to be understood that the invention is not limited to the disclosed embodiments, but, on the contrary, is intended to cover various modifications. As those skilled in the art would realize, the described embodiments may be modified in various different ways.

In the drawings, the thickness of layers, films, panels, regions, etc., are exaggerated for clarity. It will be understood that when an element such as a layer, film, region, or substrate is referred to as being "on" another element, it can be directly on the other element or intervening elements may also be present. Like reference numerals designate like elements throughout the specification.

It will be understood that, although the terms "first," "second," "third" etc. may be used herein to describe various elements, components, regions, layers and/or sections, these elements, components, regions, layers and/or sections should not be limited by these terms. These terms are only used to distinguish one element, component, region, layer or section from another element, component, region, layer or section. Thus, "a first element," "component," "region," "layer" or "section" discussed below could be termed a second element, component, region, layer or section without departing from the teachings herein.

The terminology used herein is for the purpose of describing particular embodiments only and is not intended to be limiting. As used herein, the singular forms "a," "an," and "the" are intended to include the plural forms, including "at least one," unless the context clearly indicates otherwise. "Or" means "and/or." As used herein, the term "and/or" includes any and all combinations of one or more of the associated listed items. It will be further understood that the terms "comprises" and/or "comprising," or "includes" and/or "including" when used in this specification, specify the presence of stated features, regions, integers, steps, operations, elements, and/or components, but do not preclude the presence or addition of one or more other features, regions, integers, steps, operations, elements, components, and/or groups thereof.

Furthermore, relative terms, such as "lower" or "bottom" and "upper" or "top," may be used herein to describe one element's relationship to another element as illustrated in the Figures. It will be understood that relative terms are intended to encompass different orientations of the device in addition to the orientation depicted in the Figures. For example, if the device in one of the figures is turned over, elements described as being on the "lower" side of other elements would then be oriented on "upper" sides of the other elements. The exemplary term "lower," can therefore, encompasses both an orientation of "lower" and "upper," depending on the particular orientation of the figure. Similarly, if the device in one of the figures is turned over, elements described as "below" or "beneath" other elements would then be oriented "above" the other elements. The exemplary terms "below" or "beneath" can, therefore, encompass both an orientation of above and below.

"About" or "approximately" as used herein is inclusive of the stated value and means within an acceptable range of deviation for the particular value as determined by one of ordinary skill in the art, considering the measurement in question and the error associated with measurement of the particular quantity (i.e., the limitations of the measurement system). For example, "about" can mean within one or more standard deviations, or within ± 30%, 20%, 10%, 5% of the stated value.

Unless otherwise defined, all terms (including technical and scientific terms) used herein have the same meaning as commonly understood by one of ordinary skill in the art to which this disclosure belongs. It will be further understood that terms, such as those defined in commonly used dictionaries, should be interpreted as having a meaning that is consistent with their meaning in the context of the relevant art and the present disclosure, and will not be interpreted in an idealized or overly formal sense unless expressly so defined herein.

Exemplary embodiments are described herein with reference to cross section illustrations that are schematic illustrations of idealized embodiments. As such, variations from the shapes of the illustrations as a result, for example, of manufacturing techniques and/or tolerances, are to be expected. Thus, embodiments described herein should not be construed as limited to the particular shapes of regions as illustrated herein but are to include deviations in shapes that result, for example, from manufacturing. For example, a region illustrated or described as flat may, typically, have rough and/or nonlinear features. Moreover, sharp angles that are illustrated may be rounded. Thus, the regions illustrated in the figures are schematic in nature and their shapes are not intended to illustrate the precise shape of a region and are not intended to limit the scope of the present claims.

In order to provide a liquid crystal display with a high-speed response characteristic, high transmittance, and the like, research for improving physical properties such as rotation viscosity, a refractive index, and the like of the liquid crystal composition has been conducted.

In an exemplary embodiment, a liquid crystal composition includes at least one liquid crystal molecule having positive dielectric anisotropy represented by Chemical Formula 1:

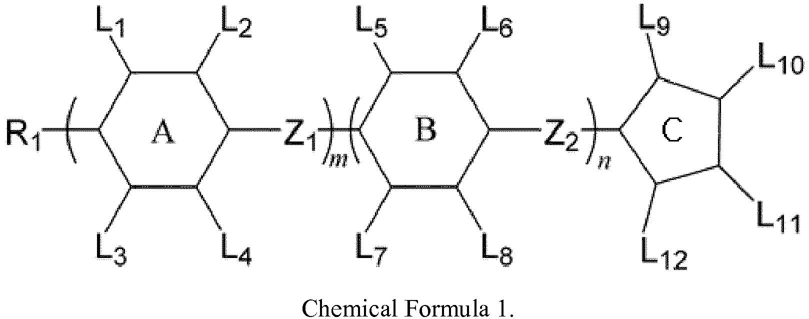

In an aspect of the exemplary embodiment, in Chemical Formula 1, each of A and B is independently 1, 4-cyclohexylene or 1, 4-phenylene, C is cyclopentyl or cyclopentadienyl, each of Z₁ and Z₂ is independently a single bond, alkylene group having the number of carbons from 1 to 10, carbonyloxy, -CF₂O-, -OCF₂-, -CH₂O or -OCH₂-,
each of L₁ to L₄ is independently dihydro group when A is 1, 4-cyclohexylene, each of L₁ to L₄ is independently -H, -F, or -CF₃ when A is 1, 4-phenylene, each of L₅ to L₈ is independently dihydro group when B is 1, 4-cyclohexylene, each of L₅ to L₉ is independently -H, -F, or -CF₃ when B is 1, 4-phenylene, each of L₉ to L₁₂ is independently dihydro group, difluoro group, or two group including one hydrogen and one fluorine when C is cyclopentyl, and each of L₉ to L₁₂ is independently -H, -F, or -CF₃ when C is cyclopentadienyl,
each of m and n is 0 to 2, provided that m and n are not simultaneously 0, and R₁ is an alkyl group or an alkoxy group.

Herein, the number of carbon atoms of R₁ may be 1 to 10.

In an exemplary embodiment, Chemical Formula 1 may include a difluoro group substituted cyclopentyl group.

In another exemplary embodiment, Chemical Formula 1 may be represented by one of Chemical Formula 1-1 to Chemical Formula 1-16:

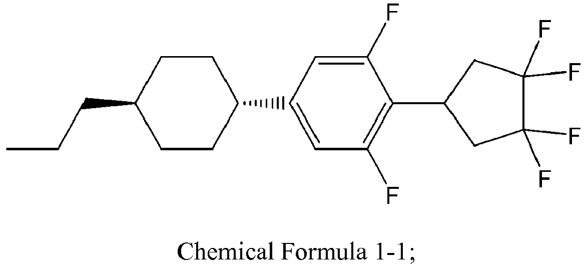

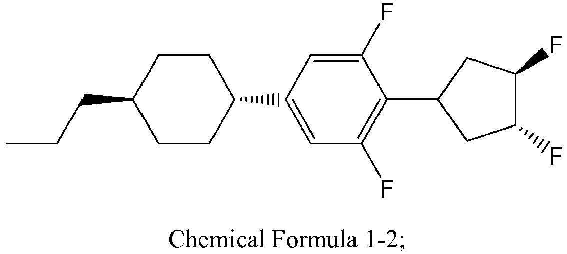

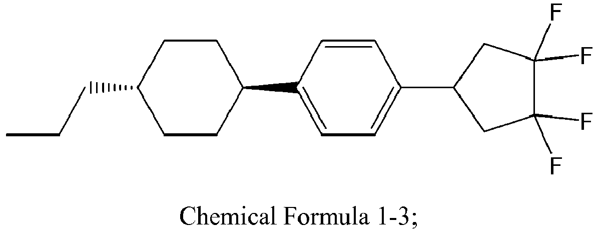

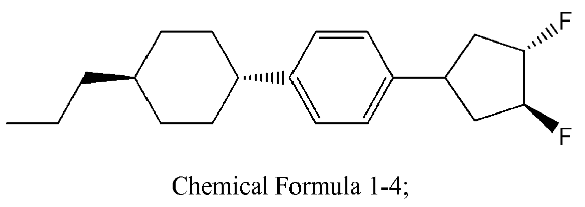

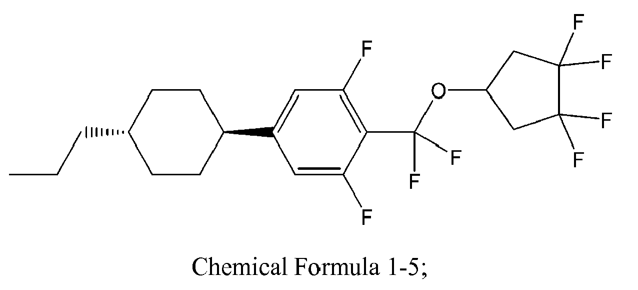

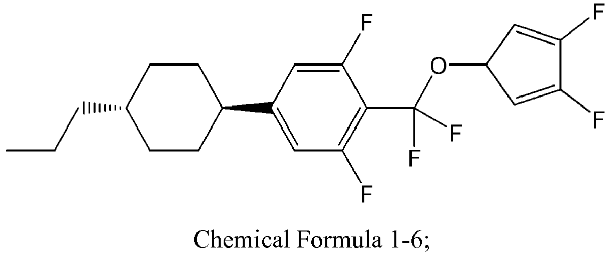

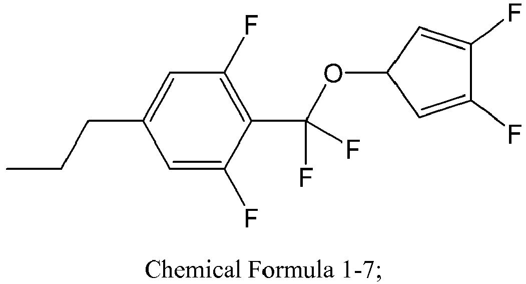

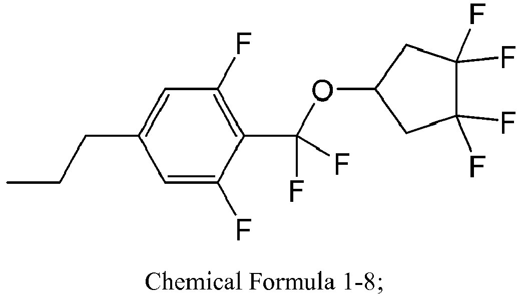

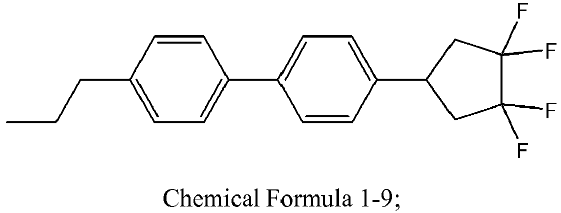

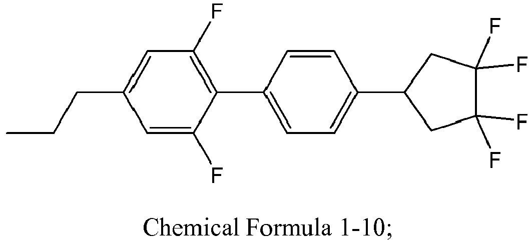

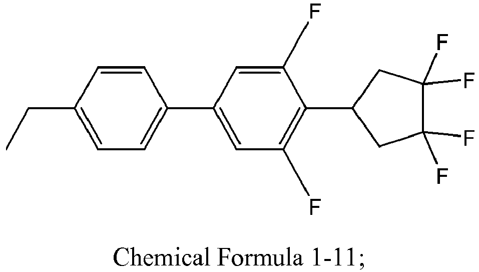

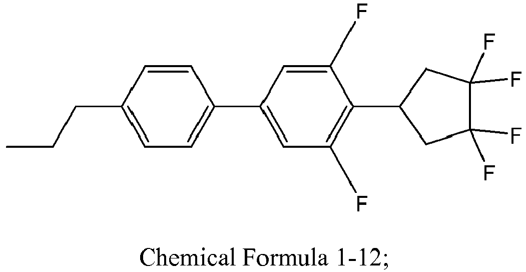

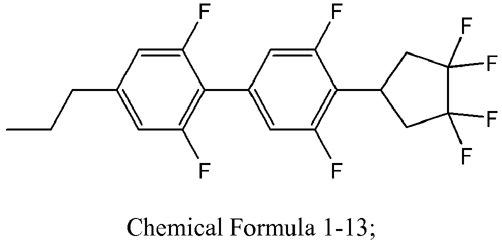

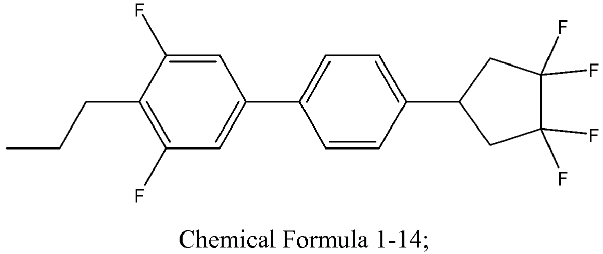

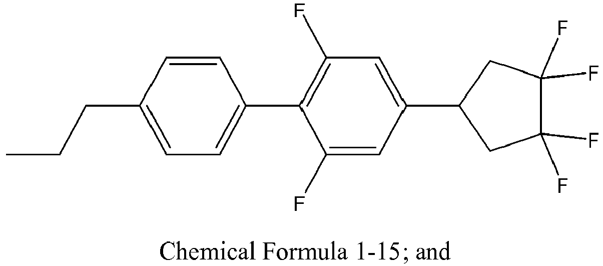

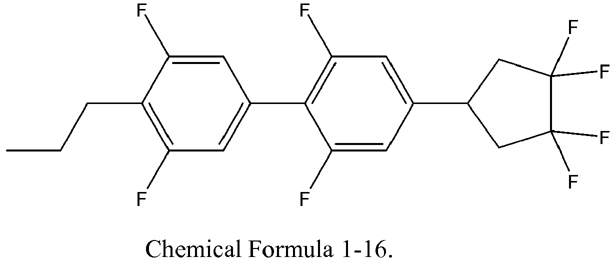

Table 1 represents physical properties, specifically the refractive anisotropy and the dielectric anisotropy of liquid crystal molecules represented by Chemical Formula 1-1 to Chemical Formula 1-16.

**Table 1**

| | Refractive anisotropy (Δn) | Dielectric anisotropy (Δε) |
|---|---|---|
| Chemical Formula 1-1 | 0.0851 | 27.63 |
| Chemical Formula 1-2 | 0.0923 | 12.61 |
| Chemical Formula 1-3 | 0.0912 | 15.843 |
| Chemical Formula 1-4 | 0.1019 | 4.8810 |
| Chemical Formula 1-5 | 0.0719 | 24.971 |
| Chemical Formula 1-6 | 0.1135 | 17.62 |
| Chemical Formula 1-7 | 0.0821 | 17.83 |
| Chemical Formula 1-8 | 0.0655 | 27.15 |
| Chemical Formula 1-9 | 0.1679 | 17.92 |
| Chemical Formula 1-10 | 0.1461 | 26.18 |
| Chemical Formula 1-11 | 0.1598 | 31.16 |
| Chemical Formula 1-12 | 0.1534 | 32.28 |
| Chemical Formula 1-13 | 0.1349 | 40.59 |
| Chemical Formula 1-14 | 0.1512 | 4.873 |
| Chemical Formula 1-15 | 0.1469 | 7.645 |
| Chemical Formula 1-16 | 0.1337 | 1.088 |

In an exemplary embodiment, among the liquid crystal molecules represented by Chemical Formula 1, liquid crystal molecules represented by Chemical Formula 1-1 may be formed based on Composition Example 1. However, the present disclosure is not limited to Composition Example 1, and the liquid crystal molecules represented by Chemical Formula 1 may be formed based on various other composition examples.

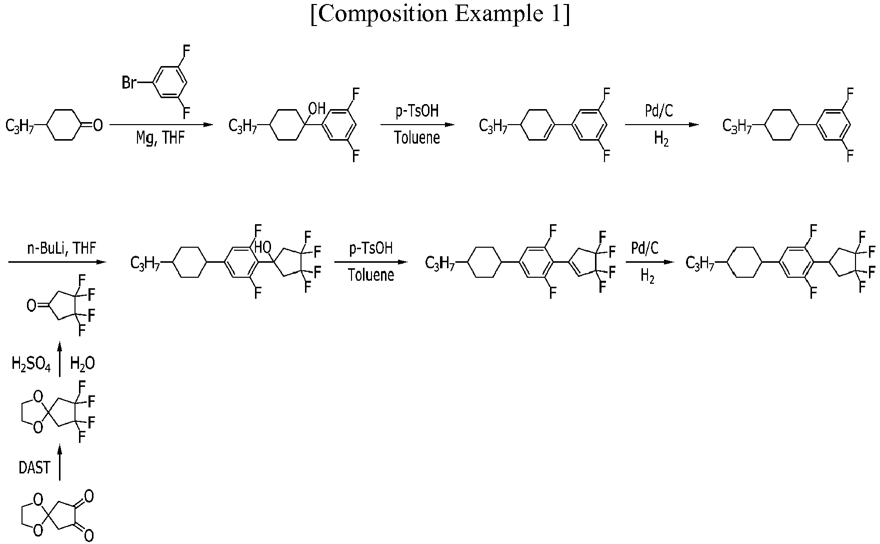

In an aspect of the exemplary embodiment, in Composition Example 1, the N,N-diethylaminosulfur trifluoride ("DAST") is employed. In the present exemplary embodiment, the DAST may be used as a reagent for fluorine substitution by deoxygenating carbonyl. However, the present disclosure is not limited thereto, and in order to substitute carbonyl with fluorine, SeF₄, DAST DeoxoFluor^{®}, and more stably XtalFluor-E^{®} and XtalFluor-M^{®} compounds in a N,N-dimethyl-1,1,2,2-tetrafluorothylamine ("TFEDMA") solid, FluoLead^{®} and the like may be used.

In another exemplary embodiment, the liquid crystal composition may further include at least one selected from a group consisting of liquid crystal molecules represented by Chemical Formula 2 and Chemical Formula 3:

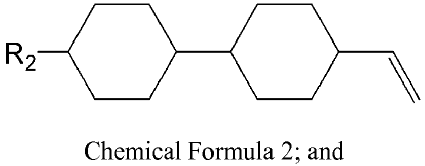

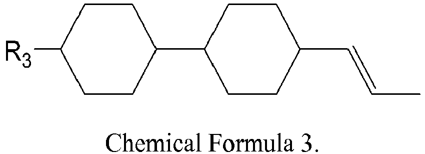

In an aspect of the exemplary embodiment, in Chemical Formula 2 and Chemical Formula 3, each of R₂ and R₃ is independently an alkyl group or an alkoxy group. Herein, the number of carbon atoms of each of R₂ and R₃ may be about 1 to about 10.

In another exemplary embodiment, the liquid crystal composition may further include at least one selected from a group consisting of liquid crystal molecules represented by Chemical Formula 4, Chemical Formula 5 and Chemical Formula 6:

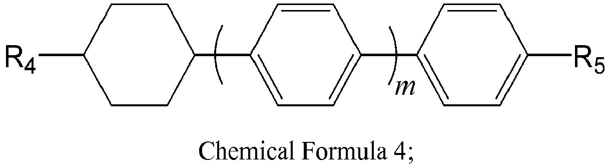

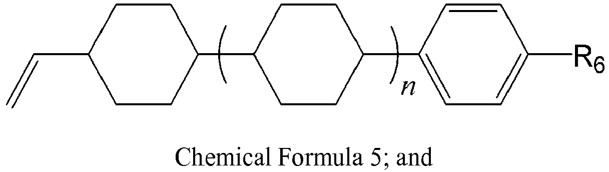

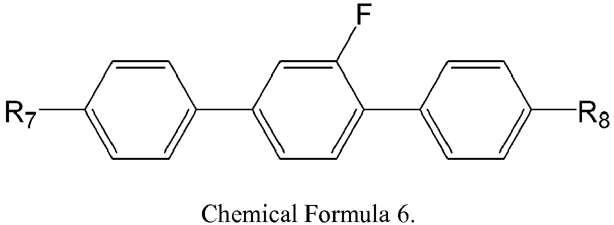

In an aspect of the exemplary embodiment, in Chemical Formula 4, Chemical Formula 5 and Chemical Formula 6, each of R₄ to R₈ is independently an alkyl group or an alkoxy group, and each of m and n is about 0 to about 2. Herein, the number of carbon atoms of each of R₄ to R₈ may be about 1 to about 10.

In another exemplary embodiment, the liquid crystal composition may further include at least one selected from a group consisting of liquid crystal molecules represented by Chemical Formula 7, Chemical Formula 8, Chemical Formula 9 and Chemical Formula 10:

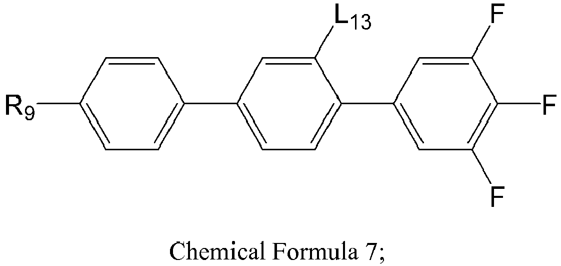

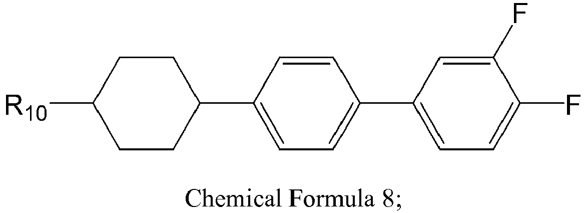

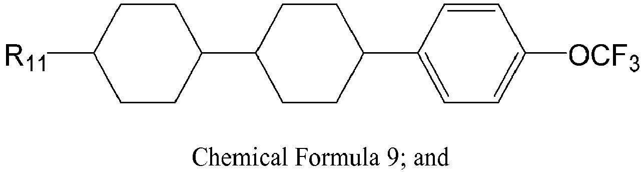

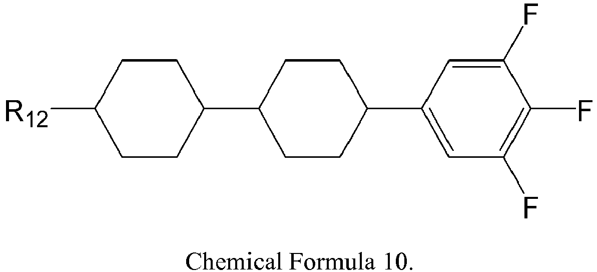

In an aspect of the exemplary embodiment, in Chemical Formula 7 to Chemical Formula 10, each of R₉ to R₁₂ is independently an alkyl group or an alkoxy group, and L₁₃ is hydrogen (H) or halogen (F, Cl, Br, I). Herein, the number of carbon atoms of each of R₉ to R₁₂ may be about 1 to about 10.

In another exemplary embodiment, the liquid crystal composition may further include at least one selected from a group consisting of liquid crystal molecules represented by Chemical Formula 11, Chemical Formula 12 and Chemical Formula 13:

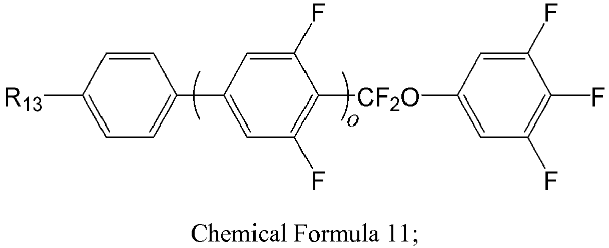

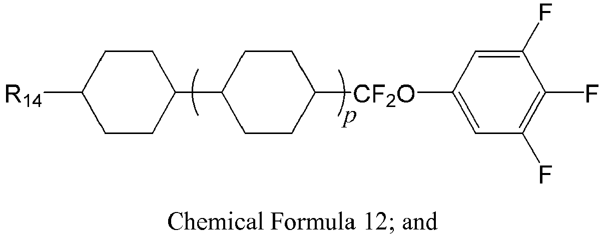

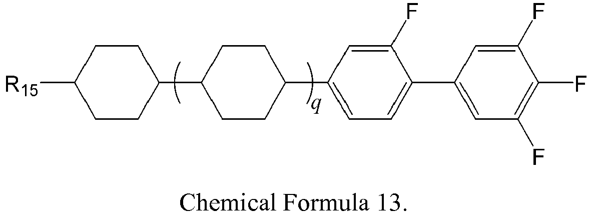

In an aspect of the exemplary embodiment, in Chemical Formula 11, Chemical Formula 12 and Chemical Formula 13, each of R₁₃ to R₁₅ is independently an alkyl group or an alkoxy group, and each of o, p, and q is about 0 to about 2. Herein, the number of carbon atoms of each of R₁₃ to R₁₅ is about 1 to about 10.

Hereinafter, a liquid crystal display formed using the above-described liquid crystal composition will be described.

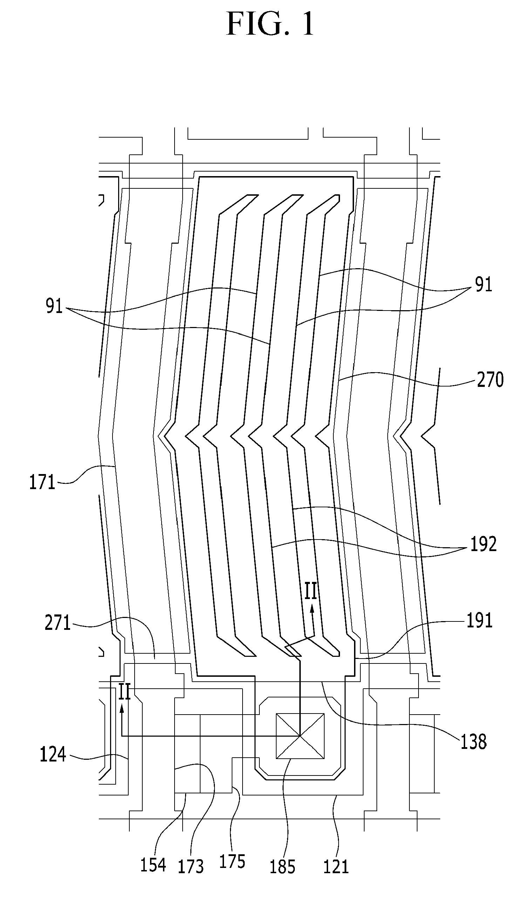

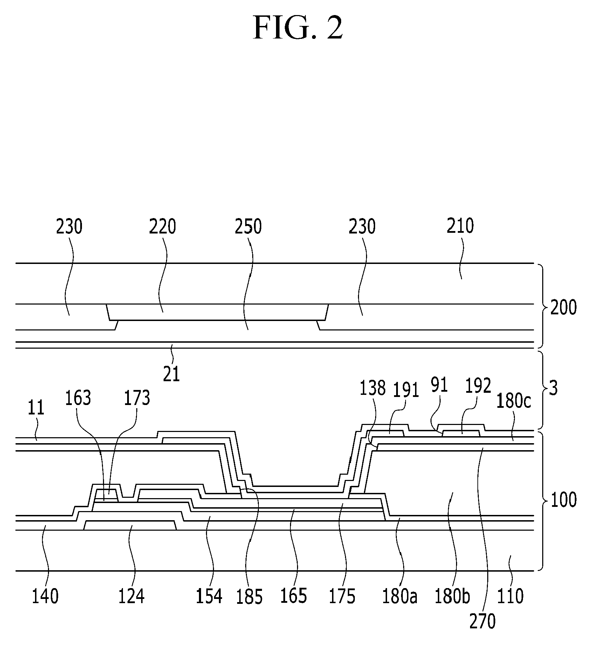

FIG. 1 illustrates a top plan view of an exemplary embodiment of the liquid crystal display. FIG. 2 illustrates a cross-sectional view of FIG. 1 taken along line II-II.

Referring to FIGS. 1 and 2, an exemplary embodiment of a liquid crystal display includes a lower panel 100 and an upper panel 200 facing each other, and a liquid crystal layer 3 interposed therebetween.

The lower panel 100 will be described first.

In an exemplary embodiment, a gate conductor including a gate line 121 is disposed on a first substrate 110 that is formed from transparent glass or plastic.

In an aspect of the exemplary embodiment, the gate line 121 includes a gate electrode 124, and a wide end portion (not shown) for connection with another layer or an external driving circuit. The gate line 121 may be made of a material including an aluminum metal such as aluminum (Al) or an aluminum alloy, a silver metal such as silver (Ag) or a silver alloy, a copper metal such as copper (Cu) or a copper alloy, a molybdenum metal such as molybdenum (Mo), or a molybdenum alloy, chromium (Cr), tantalum (Ta), titanium (Ti), etc. However, the gate line 121 may have a multilayer structure in which at least two conductive layers having different physical properties are included.

In an exemplary embodiment, a gate insulating layer 140 that is made using a silicon nitride (SiNx) or a silicon oxide (SiOx) is formed on the gate line 121. In an aspect of the exemplary embodiment, the gate insulating layer 140 may have a multilayer structure in which at least two insulating layers having different physical properties are included.

In another exemplary embodiment, a semiconductor 154 made of amorphous silicon or polysilicon is formed on the gate insulating layer 140. In an aspect of the exemplary embodiment, the semiconductor layer 154 may include an oxide semiconductor.

In an exemplary embodiment, ohmic contacts 163 and 165 are formed on semiconductor layer 154. In an aspect of the exemplary embodiment, the ohmic contacts 163 and 165 may be made from a material such as n+ hydrogenated amorphous silicon, in which an n-type impurity such as phosphorus is doped at a high concentration, or silicide. In another aspect of the exemplary embodiment, the ohmic contacts 163 and 165 may be paired to be disposed on the semiconductor layer 154. In still another aspect of the exemplary embodiment, when the semiconductor layer 154 is an oxide semiconductor, the ohmic contacts 163 and 165 may be omitted.

In an exemplary embodiment, a data line 171 including a source electrode 173 and a data conductor including a drain electrode 175 are formed on the ohmic contacts 163 and 165 and the semiconductor layer 154.

In another exemplary embodiment, the data line 171 includes a wide end portion (not shown) for connection with another layer or an external driving circuit. In an aspect of the exemplary embodiment, the data line 171 transmits a data signal and extends in a perpendicular direction to cross the gate line 121.

In an exemplary embodiment, the data line 171 may have a flexure portion having a curved shape in order to obtain maximum transmittance of a liquid crystal display, and the flexure portions may meet in a middle region of a pixel area to form a V-shape.

In still another exemplary embodiment, the source electrode 173 is part of the data line 171 and is disposed on a same line as the data line 171. In an aspect of the exemplary embodiment, the drain electrode 175 is formed to extend parallel to the source electrode 173. Therefore, the drain electrode 175 is parallel to part of the data line 171.

In an exemplary embodiment, a gate electrode 124, a source electrode 173 and a drain electrode 175 form one thin film transistor ("TFT") with a semiconductor layer 154, and a channel of the thin film transistor is formed on the semiconductor layer 154 between the source electrode 173 and the drain electrode 175.

In an exemplary embodiment, the liquid crystal display includes a source electrode 173 disposed on the same line as the data line 171, and a drain electrode 175 stretched parallel to the data line 171, thereby broadening the width of the thin film transistor without widening the area occupied by a data conductor, and thus increasing the aperture ratio of the liquid crystal display.

In an exemplary embodiment, the data line 171 and the drain electrode 175 are made of a refractory metal such as molybdenum, chromium, tantalum, and titanium, or an alloy thereof, and may have a multilayer structure including a refractory metal layer (not shown) and a low resistance conductive layer (not shown). Non-limiting examples of the multilayer structure include a double layer of a chromium or chromium alloy or molybdenum, or molybdenum alloy lower layer and an aluminum or aluminum alloy) upper layer, and a triple layer of a molybdenum or molybdenum alloy) lower layer, an aluminum or aluminum alloy intermediate layer, and a molybdenum or molybdenum alloy upper layer.

In an exemplary embodiment, a first passivation layer 180a is disposed on exposed portions of the data conductors 171, 173 and 175, the gate insulating layer 140, and the semiconductor 154. The first passivation layer 180a may be formed using an organic insulating material or an inorganic insulating material.

In another exemplary embodiment, a second passivation layer 180b is formed on the first passivation layer 180a. The second passivation layer 180b may be formed using an organic insulator.

In still another exemplary embodiment, the second passivation layer 180b may be a color filter. When the second passivation layer 180b is a color filter, the second passivation layer 180b may inherently display one primary color, and examples of primary colors include red, green, blue, yellow, cyan, magenta, or the like. Though not shown herein, a color filter displaying a mixture of the primary colors, or white, in addition to the primary colors, may be further included as the color filter. When the second passivation layer 180b is a color filter, the color filter 230 may be omitted from an upper panel 200 to be described below. In another exemplary embodiment, the second passivation layer 180b may be formed from an organic insulating material, and a color filter (not shown) may be formed between the first passivation layer 180a and the second passivation layer 180b.

In an exemplary embodiment, a common electrode 270 is provided on the second passivation layer 180b. The common electrode 270, having a planar shape, may be formed on a front surface of a substrate 110 as one plate, and it may have an opening 138 disposed in a region corresponding to the surrounding of the drain electrode 175. The opening 138 may extend parallel to the gate line 121, and it may be formed as a separate shape in a portion overlapping the data line 171. That is, in the present exemplary embodiment, the common electrodes 270 are not separated, but are integrally connected at an area corresponding to a unit pixel in order to have a one-plate shape. In this case, the common electrodes 270 of pixels adjacent to each other in a direction of the gate line 121 may be connected to each other through a connector 271.

In an exemplary embodiment, the common electrodes 270 disposed in adjacent pixels are connected to each other, and may receive a certain level of common voltage transmitted from outside of a display area.

In an exemplary embodiment, an insulating layer 180c is disposed on the common electrode 270. In an aspect of the exemplary embodiment, the insulating layer 180c may be formed from an organic insulating material or an inorganic insulating material.

In an exemplary embodiment, a pixel electrode 191 is disposed on the insulating layer 180c. In an aspect of the exemplary embodiment, the pixel electrode 191 includes a curved edge substantially parallel to the flexure portion of a data line 171. In another aspect of the exemplary embodiment, the pixel electrode 191 has a plurality of cutouts 91 and includes a plurality of branch electrodes 192 disposed between neighboring cutouts 91. The plurality of branch electrodes 192 overlaps the common electrodes 270 in a plain view.

In an exemplary embodiment, the pixel electrode 191 is a first field generating electrode or a first electrode, and the common electrode 270 is a second field generating electrode or a second electrode. In an aspect of the exemplary embodiment, the pixel electrode 191 and the common electrode 270 may form a fringe field.

In another exemplary embodiment, a contact hole 185 exposing the drain electrode 175 is formed in the first passivation layer 180a, the second passivation layer 180b, and the insulating layer 180c. In an aspect of the exemplary embodiment, the pixel electrode 191 is physically and electrically connected to the drain electrode 175 through a contact hole 185, and receives a voltage from the drain electrode 175.

In another exemplary embodiment, a first alignment layer 11 is formed on the pixel electrode 191 and the insulating layer 180c

In an aspect of the exemplary embodiment, the first alignment layer 11 may be a horizontal alignment layer and may be rubbed in a predetermined direction. However, the first alignment layer 11 is not limited to a rubbing alignment layer, and it may be formed as a photo-alignment layer.

The upper panel 200 will now be described.

In an exemplary embodiment, a light blocking member 220, a plurality of color filters 230, an overcoat 250, and a second alignment layer 21 are disposed between a second substrate 210 and a liquid crystal layer 3. The second substrate 210 is made of transparent glass, plastic, or the like. The light blocking member 220 is called a black matrix and prevents light leakage.

In another exemplary embodiment, the plurality of color filters 230 are is disposed between the second substrate 210 and the overcoat 250. An edge of the color filter 230 may cover the light blocking member 220. In an aspect of the exemplary embodiment, when the second passivation layer 180b of the lower panel 100 is a color filter, or a color filter is formed on the lower panel 100, the color filter 230 of the upper panel 200 may be omitted. In another aspect of the exemplary embodiment, the light blocking member 220 of the upper panel 200 may be formed on the lower panel 100.

In an exemplary embodiment, the overcoat 250 is disposed between the color filter 230 and the second alignment layer 21, and between the light blocking member 220 and the second alignment layer 21. In an aspect of the exemplary embodiment, the overcoat 250 may be made of an (organic) insulating material, and it prevents the color filter 230 from being exposed and provides a flat surface. In another aspect of the exemplary embodiment, the overcoat 250 may be omitted.

In an exemplary embodiment, the second alignment layer 21 is disposed between the overcoat 250 and a liquid crystal layer 3. In an aspect of the exemplary embodiment, the second alignment layer 21 may be formed from the same material as the above-described first alignment layer 11.

In an exemplary embodiment, a liquid crystal layer 3 is provided between the lower panel 100 and the upper panel 200. In an aspect of the exemplary embodiment, the liquid crystal layer 3 includes at least one liquid crystal molecule that includes at least one difluoro (-diF) group substituted cyclopentyl group, and more specifically, the above-described contents on the liquid crystal composition may be applicable to the liquid crystal layer 3.

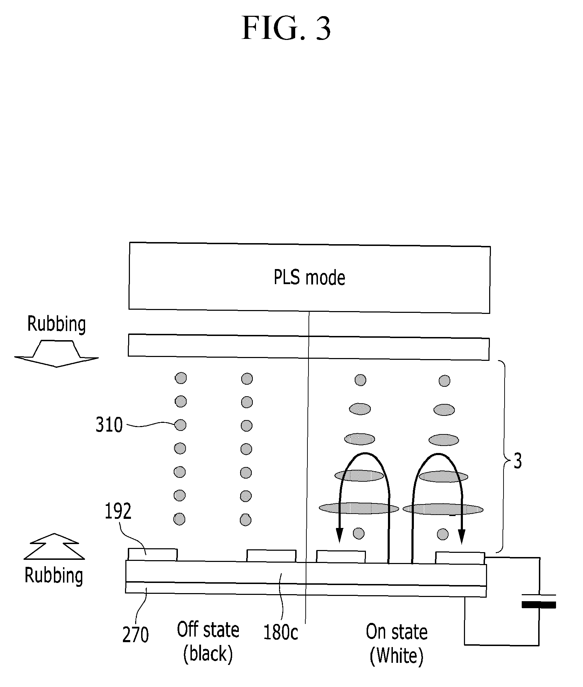

FIG. 3 illustrates a schematic cross-sectional view illustrating of an exemplary embodiment of an alignment of liquid crystal molecules depending on whether an electric field is applied in a liquid crystal display.

Referring to FIG. 3, in an exemplary embodiment, the first alignment layer 11 and the second alignment layer 21 may be rubbed in a direction parallel to the plurality of branch electrodes 192. In another exemplary embodiment, when the first alignment layer 11 and the second alignment layer 21 are formed of a photo-alignment material, surfaces of the first alignment layer 11 and the second alignment layer 21 may be photo-aligned in a direction parallel to the plurality of branch electrodes 192.

In an exemplary embodiment, the liquid crystal molecules 310 of the liquid crystal layer 3 may be aligned so that long axes thereof are parallel to the display panels 100 and 200. Particularly, in an aspect of the exemplary embodiment, the long axes of the liquid crystal molecules 310 may extend in a direction parallel to the plurality of branch electrodes 192 in an off state in which an electric field is not applied to the liquid crystal molecules 310. In other words, the liquid crystal molecules 310 tilt in a direction in which the branch electrodes 192 are extended.

In another exemplary embodiment, the long axes of the liquid crystal molecules 310 according to the present exemplary embodiment may be aligned to be parallel to an electric field direction in a state in which the electric field is applied to the liquid crystal molecules 310. In an aspect of the exemplary embodiment, the liquid crystal display is a positive type in-plane switching mode liquid crystal display, and thus the liquid crystal layer 3 may be formed of a liquid crystal material in which the entire liquid crystal composition has a positive dielectric anisotropy. Particularly, in an aspect of the exemplary embodiment, the liquid crystal display shown in FIG. 3 is planar in order to line the switching mode liquid crystal display in which a plane shape of field generating electrode and a linear shape of field generating electrode form the electric field of the liquid crystal layer 3 with the insulating layer therebetween.

Referring to FIGS. 1 and 2, in an exemplary embodiment, the pixel electrode 191 receives a data voltage from the drain electrode 175, and the common electrode 270 receives a common voltage from a common voltage applier provided outside of the display area.

In an exemplary embodiment, the pixel electrode 191, which is a field generating electrode, and the common electrode 270 generate an electric field, such that the liquid crystal molecules of the liquid crystal layer 3 disposed on these field generating electrodes 191 and 270 may rotate in a direction perpendicular or parallel to the generated electric field. In an aspect of the exemplary embodiment, depending on the rotation direction of the liquid crystal molecules determined as described above, polarization of light transmitted through the liquid crystal layer is changed.

As described above, in an exemplary embodiment, by forming two field generating electrodes 191 and 270 on one display panel 100, the transmittance of the liquid crystal display may be improved and a wide viewing angle may be realized.

In another exemplary embodiment of the liquid crystal display as shown, although the common electrode 270 has a plane shape and the pixel electrode 191 has a plurality of branch electrodes, in another exemplary embodiment, a liquid crystal display may include a pixel electrode 191 having a plane shape and a common electrode 270 having a plurality of branch electrodes.

The present disclosure may be applied to all other cases where two field generating electrodes overlap an insulating layer interposed therebetween on a first substrate 110, a first field generating electrode formed under the insulating layer has a planar shape, and a second field generating electrode formed on the insulating layer has a plurality of branch electrodes.

In an exemplary embodiment, the polarizers (not shown) may be provided on the outer surfaces of the two display panels 100 and 200, and transmissive axes of the two polarizers are perpendicular to each other and one of the transmissive axes may be parallel to the gate line 121. In another exemplary embodiment, in the case of a reflective liquid crystal display, one of the two polarizers may be omitted.

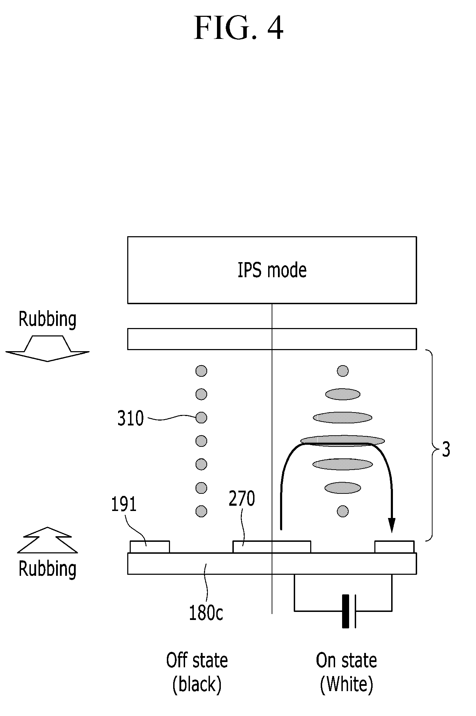

FIG. 4 illustrates a schematic cross-sectional view of an exemplary embodiment of an in-plane switching mode liquid crystal display as a variation of FIG. 3.

Unlike the plane to line switching mode liquid crystal display described in FIG. 3, the exemplary embodiment of the liquid crystal display shown in FIG. 4 is a positive type in-plane switching mode liquid crystal display in which both the pixel electrode 191 and the common electrode 270 are formed to have a linear shape field generating electrode. Similar to the exemplary embodiment of FIG. 3, since the exemplary embodiment of FIG. 4 generates a horizontal electric field and an alignment direction of an alignment layer is parallel to a linear shape of the electrode, a liquid crystal display may be formed by using the above-described exemplary embodiment of the liquid crystal composition.

An exemplary embodiment of a liquid crystal composition and the physical properties thereof will now be described.

### Exemplary Embodiment 1

**Table 2**

| Liquid crystal composition | Content (weight percent) |
|---|---|
| 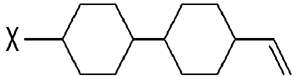 | 28 |
| 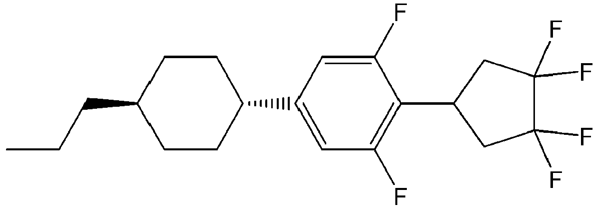 | 17 |
| 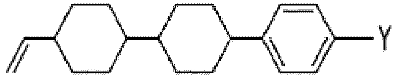 | 10 |
| 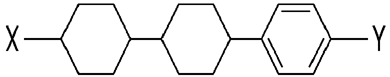 | 8 |
| 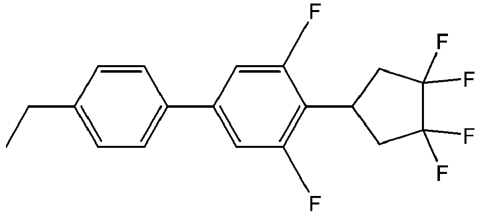 | 13 |
| 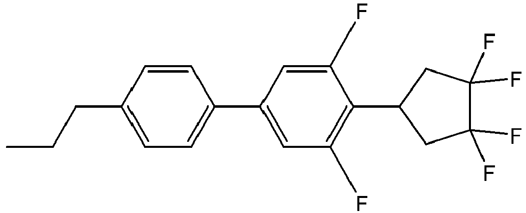 | 14 |
| 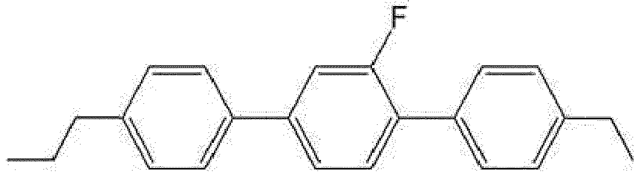 | 10 |

In Table 2, each of X and Y independently includes an alkyl group having 1 to 5 carbon atoms. Exemplary Embodiment 1 includes liquid crystal molecule represented by Chemical Formula 1-1, Chemical Formula 1-11 and Chemical Formula 1-12 as described above with regard to the exemplary embodiments of the liquid crystal composition.

According to experimental results of the physical properties of the liquid crystal composition of Exemplary Embodiment 1, the phase transition temperature (Tni) thereof is about 78.3 degrees Celsius, the refractive anisotropy (Δn) is about 0.1093, the rotation viscosity (γ1) is about 55.7 millipascal seconds (mPa·s), and the dielectric anisotropy (Δε) is about 4.57. Thus, the exemplary embodiment(s) of the liquid crystal composition exhibit substantially comparable physical properties as those exhibited by conventional liquid crystal compositions.

While this invention has been described in connection with what is presently considered to be practical exemplary embodiments, it is to be understood that the invention is not limited to the disclosed embodiments, but, on the contrary, is intended to cover various modifications and equivalent arrangements included within scope of the appended claims.

## Claims

1. A liquid crystal composition comprising at least one liquid crystal molecule (310) having a positive dielectric anisotropy represented by Chemical Formula 1: 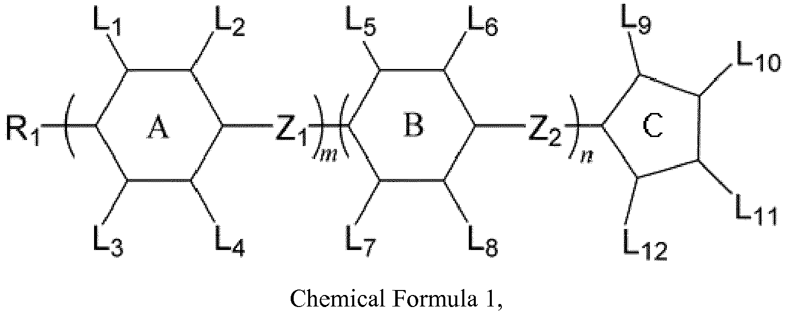
wherein, in Chemical Formula 1, each of A and B is independently 1, 4-cyclohexylene or 1, 4-phenylene, C is cyclopentyl or cyclopentadienyl, and each of Z₁ and Z₂ is independently a single bond, alkylene group having the number of carbons from 1 to 10, carbonyloxy, -CF₂O-, -OCF₂-, -CH₂O-, or -OCH₂-,
wherein each of L₁ to L₄ is independently dihydro group when A is 1, 4-cyclohexylene, each of L₁ to L₄ is independently -H, -F, or -CF₃ when A is 1, 4-phenylene, each of L₅ to L₈ is independently dihydro group when B is 1, 4-cyclohexylene, each of L₅ to L₈ is independently -H, -F, or -CF₃ when B is 1, 4-phenylene, each of L₉ to L₁₂ is independently dihydro group, difluoro group, or two group including one hydrogen and one fluorine when C is cyclopentyl, and each of L₉ to L₁₂ is independently -H, -F, or -CF₃ when C is cyclopentadienyl,
wherein each of m and n is 0 to 2, provided that m and n are not simultaneously 0, and R₁ is an alkyl group or an alkoxy group, and the number of carbons of R₁ is about 1 to about 10.

2. The liquid crystal composition of claim 1, wherein
Chemical Formula 1 is represented by at least one of Chemical Formulas 1-1 to Chemical Formula 1-16: 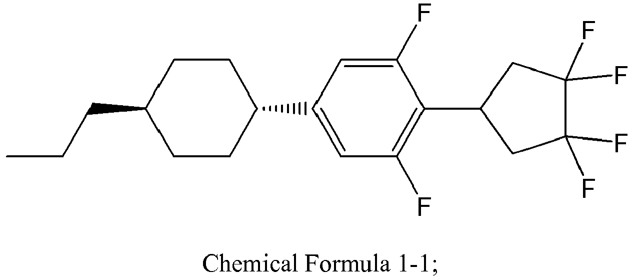 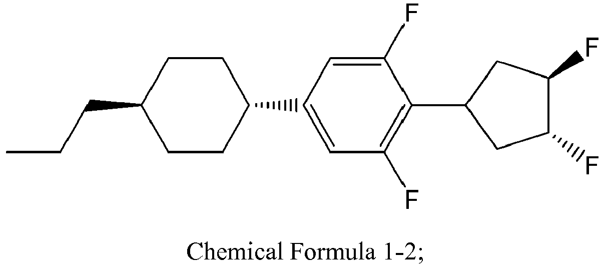 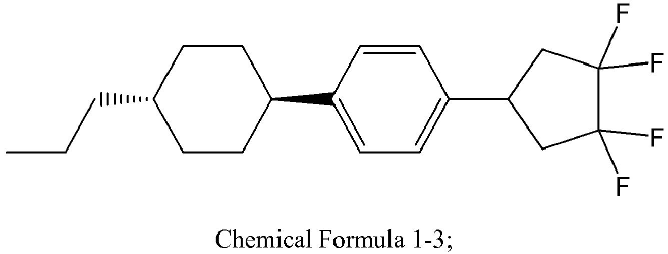 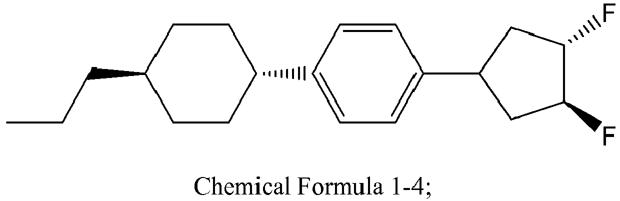 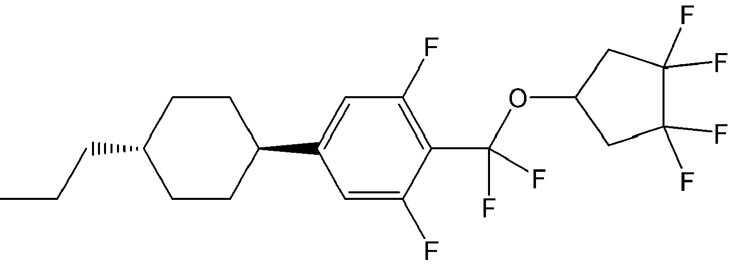 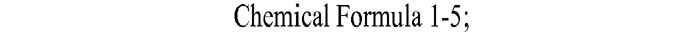 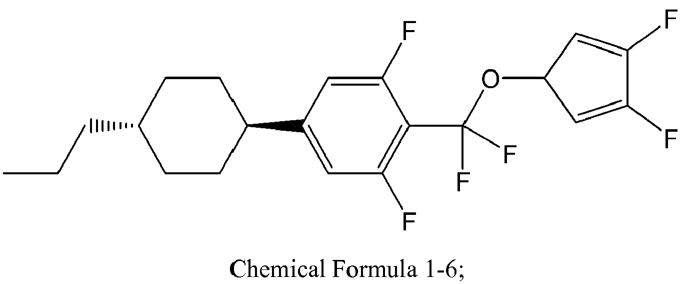 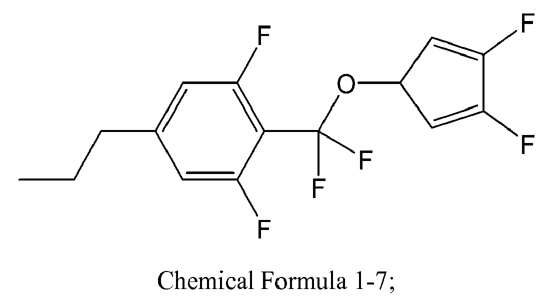 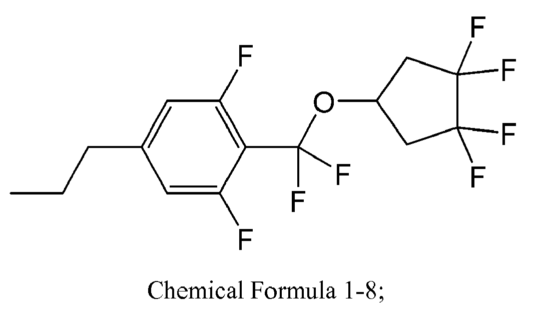 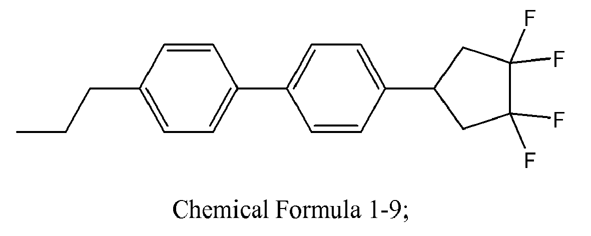 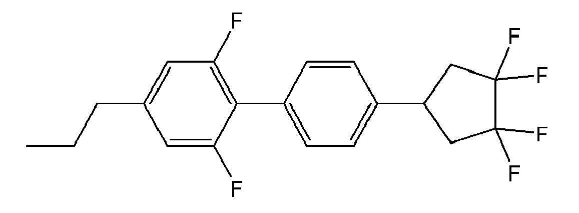 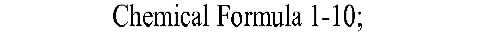 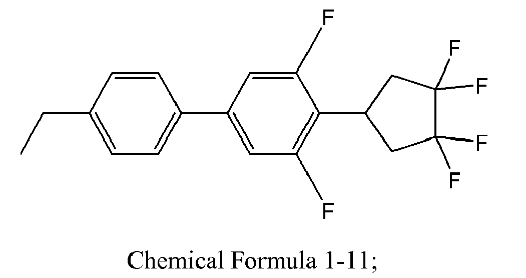 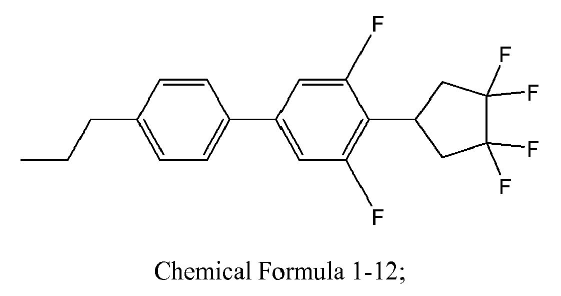 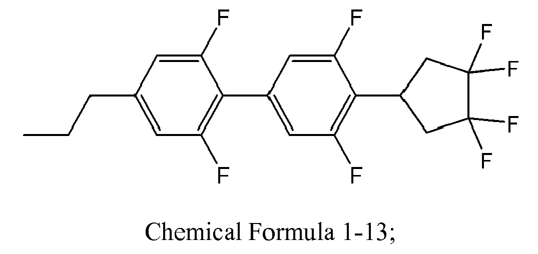 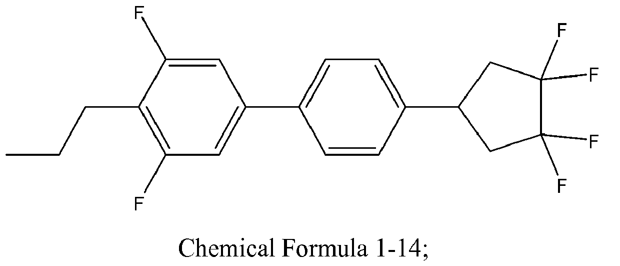 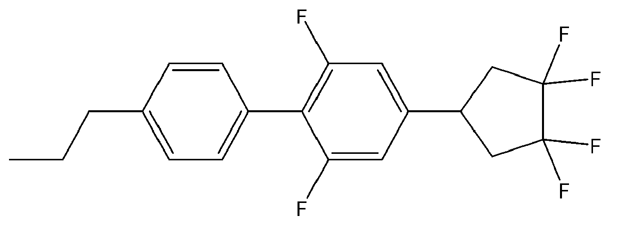 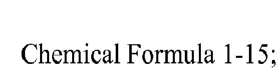 and

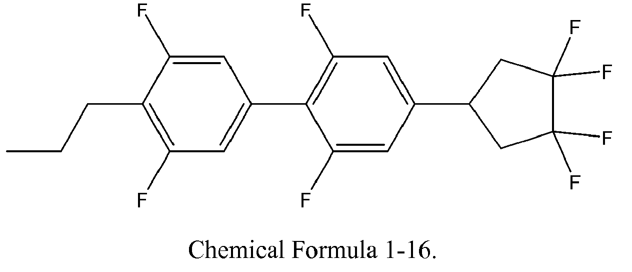

3. The liquid crystal composition of claim 2, further comprising at least one selected from a group consisting of liquid crystal molecule (310) represented by Chemical Formula 2 and Chemical Formula 3: 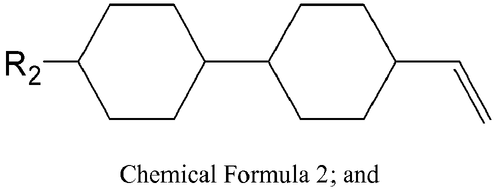 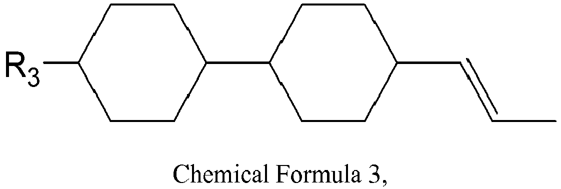 wherein, in Chemical Formula 2 and Chemical Formula 3, each of R₂ and R₃ is independently an alkyl group or an alkoxy group, and the number of carbons of each of R₂ and R₃ is about 1 to about 10.

4. The liquid crystal composition of claim 3, further comprising
at least one selected from a group consisting of liquid crystal molecules (310) represented by Chemical Formula 4, Chemical Formula 5 and Chemical Formula 6: 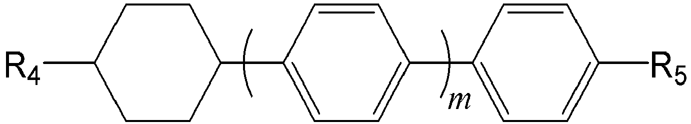 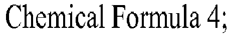 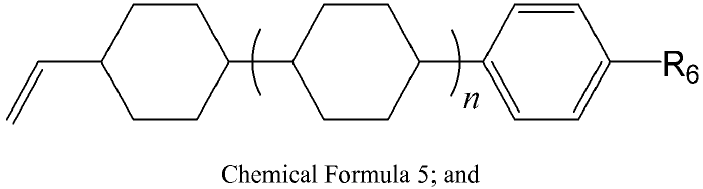 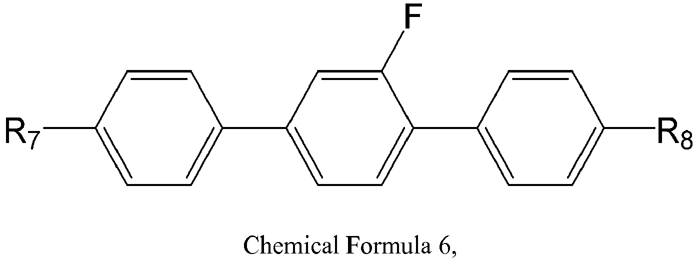 wherein, in Chemical Formula 4, Chemical Formula 5, and Chemical Formula 6, each of R₄ to R₈ is independently an alkyl group or an alkoxy group, and each of m and n is about 0 to about 2, and the number of carbons of each of R₄ to R₈ is about 1 to about 10.

5. The liquid crystal composition of claim 4, further comprising
at least one selected from a group consisting of liquid crystal molecules (310) represented by Chemical Formula 7, Chemical Formula 8, Chemical Formula 9 and Chemical Formula 10: 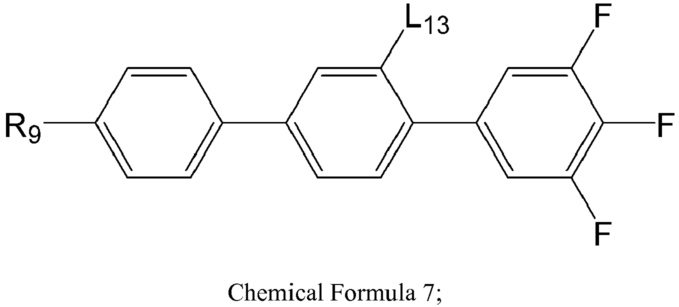 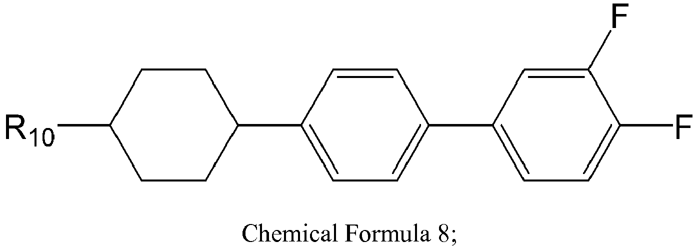 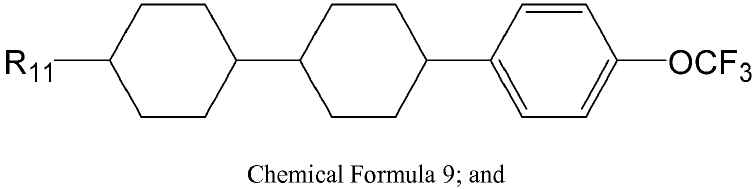 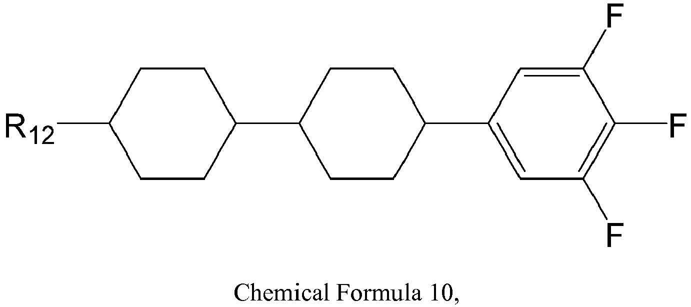 wherein, in Chemical Formula 7 to Chemical Formula 10, each of R₉ to R₁₂ is independently an alkyl group or an alkoxy group, and L₁₃ is hydrogen or halogen, and the number of carbons of each of R₉ to R₁₂ is about 1 to about 10.

6. A liquid crystal display comprising:
a first substrate (110);
a first electrode and a second electrode disposed on the first substrate (110) with an insulating layer (180c) therebetween;
a second substrate (210) facing the first substrate (110); and
a liquid crystal layer (3) disposed between the first substrate (110) and the second substrate (210),
wherein the liquid crystal layer (3) comprises at least one liquid crystal molecule (310) having a positive dielectric anisotropy represented by Chemical Formula 1: 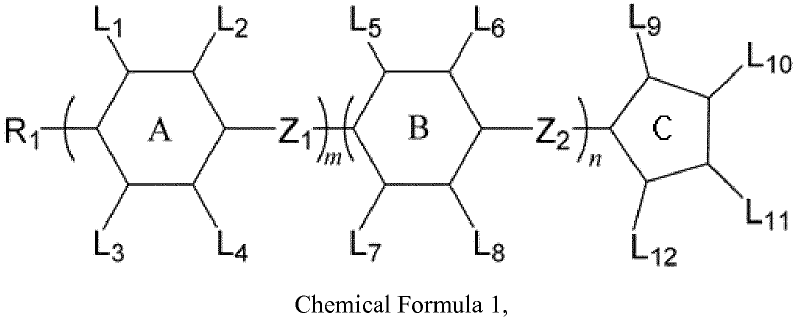
wherein, in Chemical Formula 1, each of A and B is independently 1 4-cyclohexylene or 1, 4-phenylene, C is cyclopentyl or cyclopentadienyl, and each of Z and Z₂ is independently a single bond, alkylene group having the number of carbons from 1 to 10, carbonyloxy, -CF₂O-, -OCF₂-, -CH₂O-, or -OCH₂-,
wherein each of L₁ to L₄ is independently dihydro group when A is 1 4-cyclohexylene, each of L₁ to L₄ is independently -H, -F, or -CF₃ when A is 1 4-phenylene, each of L₅ to L₈ is independently dihydro group when B is 1 4-cyclohexylene, each of L₅ to L₈ is independently -H, -F, or -CF₃ when B is 1 4-phenylene, each of L₉ to L₁₂ is independently dihydro group, difluoro group, or two group including one hydrogen and one fluorine when C is cyclopentyl, and each of L₉ to L₁₂ is independently -H, -F, or -CF₃ when C is cyclopentadienyl,
wherein each of m and n is 0 to 2, provided that m and n are not simultaneously 0, and R₁ is an alkyl group or an alkoxy group, and the number of carbons of R₁ is about 1 to about 10.

7. The liquid crystal display of claim 6, wherein
Chemical Formula 1 is represented by at least one of Chemical Formulas 1-1 to Chemical Formula 1-16: 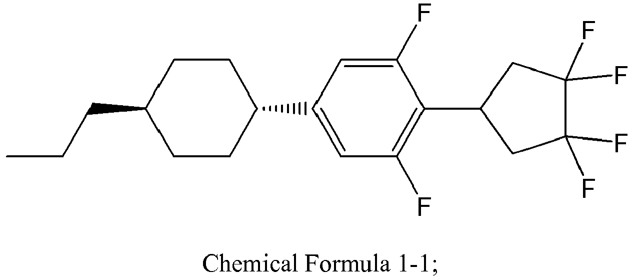 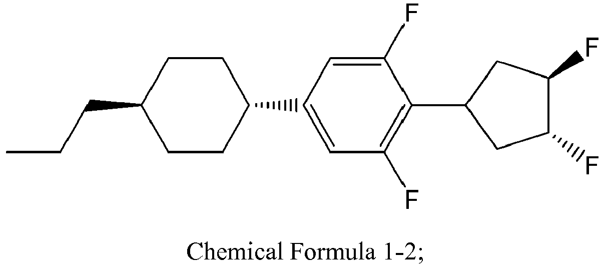 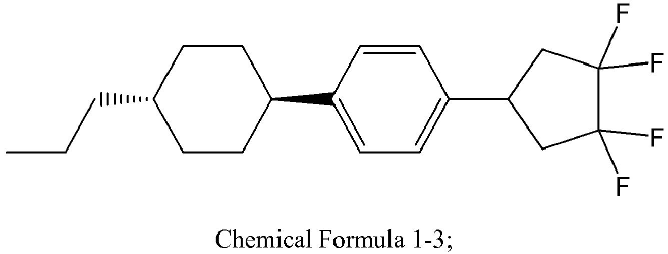 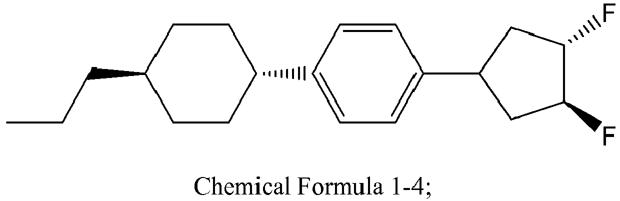 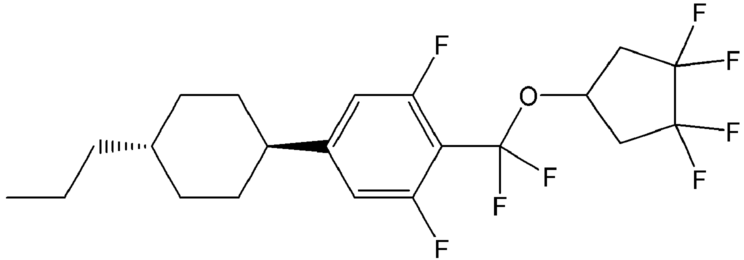 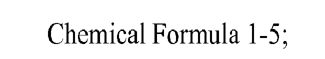 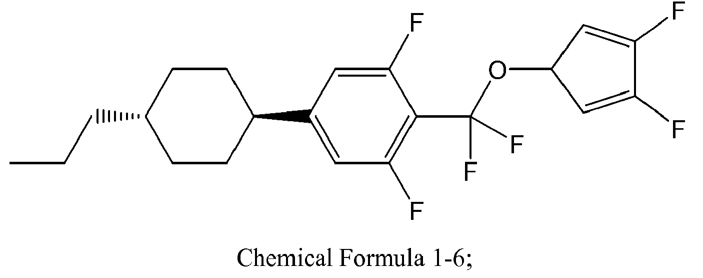 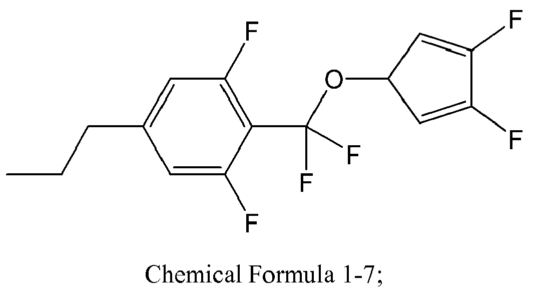 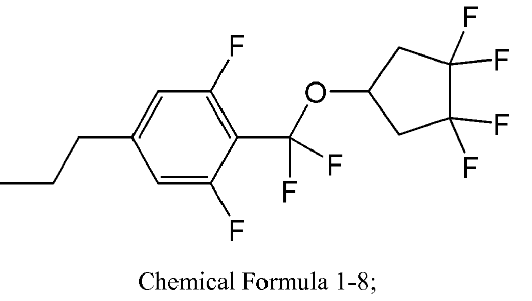 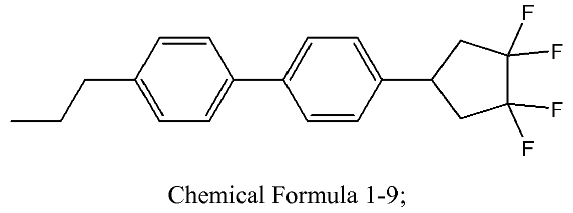 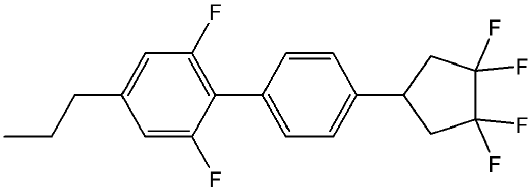 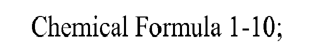 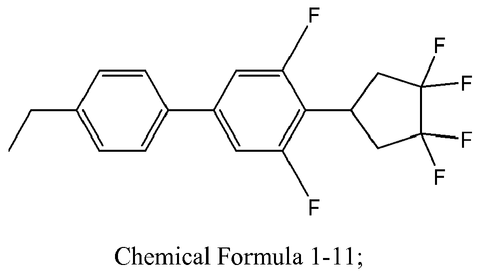 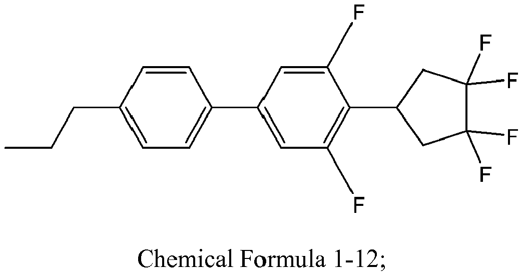 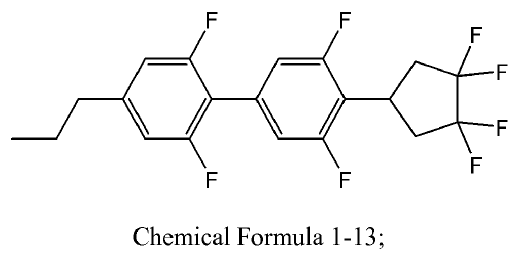 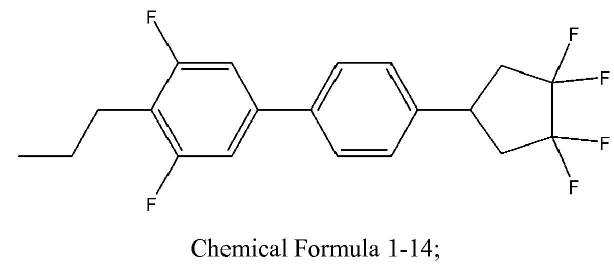 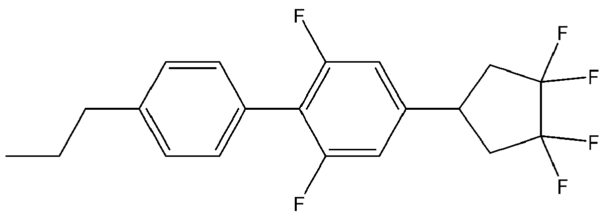 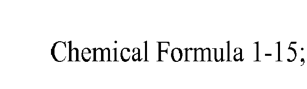 and

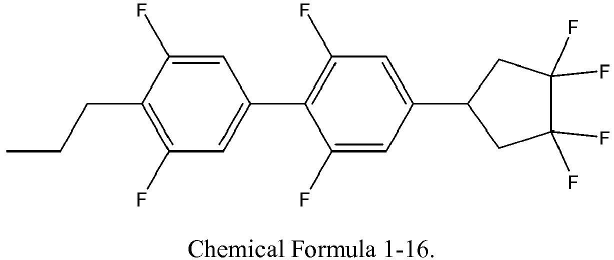

8. The liquid crystal display of claim 7, further comprising
at least one selected from a group consisting of liquid crystal molecules (310) represented by Chemical Formula 2 and Chemical Formula 3: 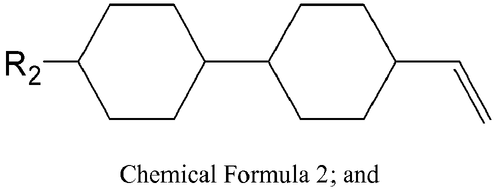 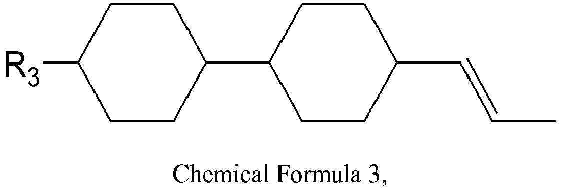 wherein, in Chemical Formula 2 and Chemical Formula 3, each of R₂ and R₃ is independently an alkyl group or an alkoxy group, and the number of carbons of each of R₂ and R₃ is about 1 to about 10.

9. The liquid crystal display of claim 8, further comprising
at least one selected from a group consisting of liquid crystal molecules (310) represented by Chemical Formula 4, Chemical Formula 5 and Chemical Formula 6: 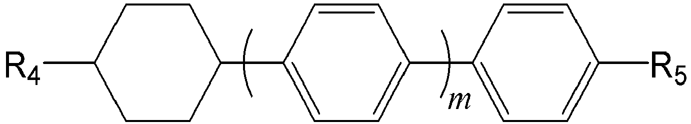 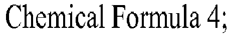 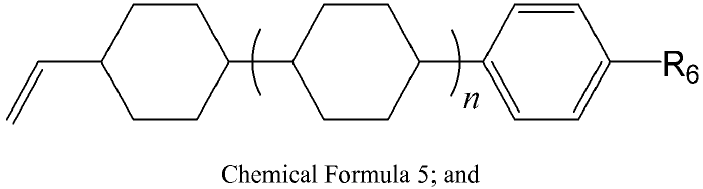 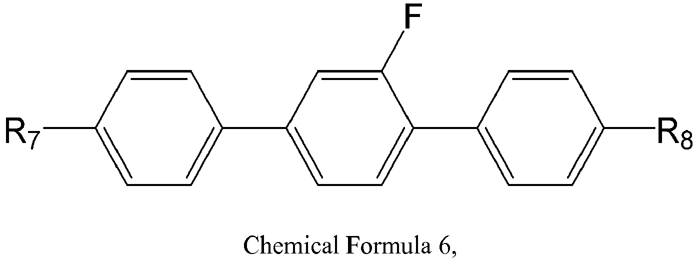 wherein, in Chemical Formula 4, Chemical Formula 5 and Chemical Formula 6, each of R₄ to R₈ is independently an alkyl group or an alkoxy group, and each of m and n is about 0 to about 2, and the number of carbons of each of R₄ to R₈ is about 1 to about 10.

10. The liquid crystal display of claim 9, further comprising
at least one selected from a group consisting of liquid crystal molecules (310) represented by Chemical Formula 7, Chemical Formula 8, Chemical Formula 9 and Chemical Formula 10: 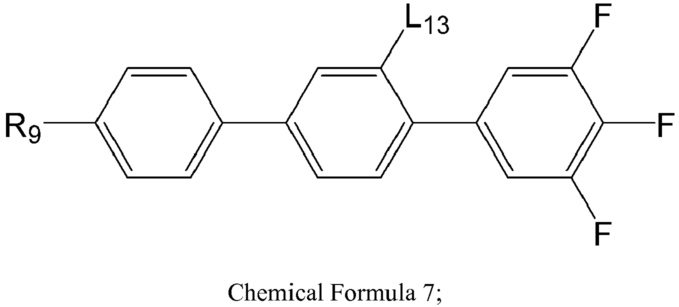 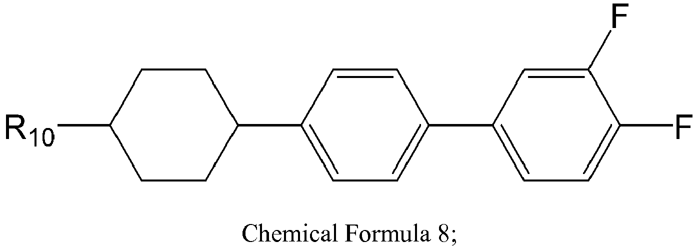 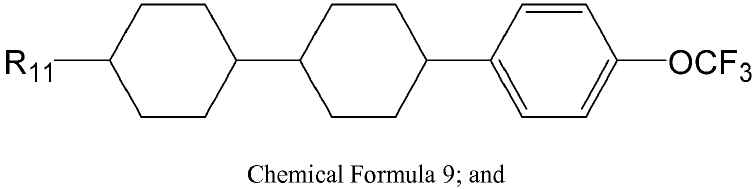 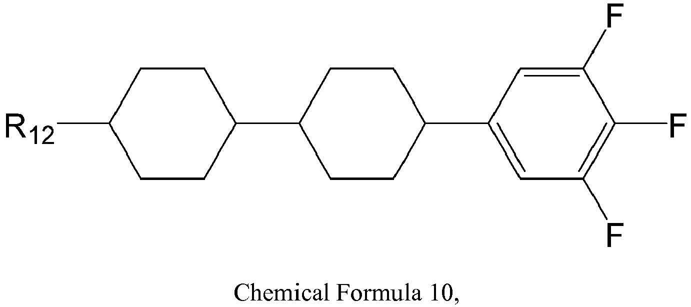 wherein, in Chemical Formula 7 to Chemical Formula 10, each of R₉ to R₁₂ is independently an alkyl group or an alkoxy group, and L₁₃ is hydrogen or halogen, and the number of carbons of each of R₉ to R₁₂ is about 1 to about 10.

11. The liquid crystal display of claim 10, further comprising
at least one selected from a group consisting of liquid crystal molecules (310) represented by Chemical Formula 11, Chemical Formula 12 and Chemical Formula 13: 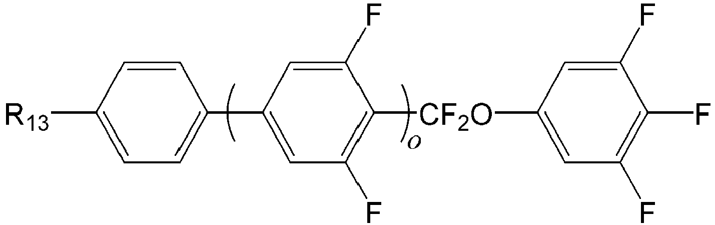 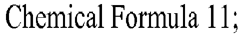 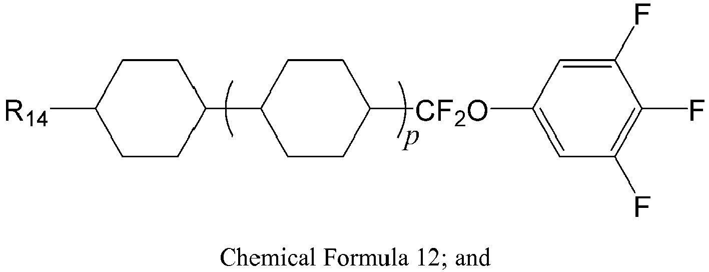 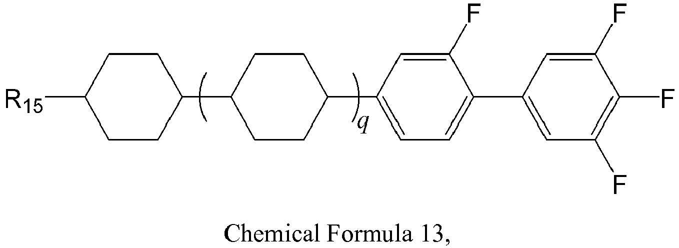 wherein, in Chemical Formula 11, Chemical Formula 12, and Chemical Formula 13, each of R₁₃ to R₁₅ is independently an alkyl group or an alkoxy group, and each of o, p, q is about 0 to about 2, and the number of carbons of each of R₁₃ to R₁₅ is about 1 to about 10.

12. The liquid crystal display of one of claims 6 to 11, wherein
the insulating layer (180c) is disposed on the first electrode, the second electrode is disposed on the insulating layer (180c),
the first electrode is formed to have a one-plate shape, and the second electrode comprises a plurality of branch electrodes (192).

13. The liquid crystal display of claim 12, wherein
liquid crystal molecules (310) in the liquid crystal layer (3) tilt in a direction parallel to the branch electrodes (192) when an electric field is not applied to the liquid crystal layer (3).

14. The liquid crystal display of claim 12 or 13, wherein
the liquid crystal molecules (310) tilt in a direction parallel to an electric field when the electric field is applied to the liquid crystal layer (3).

15. The liquid crystal display of one of claims 12 to 14, wherein the first electrode is formed to have a one-plate shape at an area corresponding to a unit pixel.
